(19) Europäisches Patentamt
European Patent Office
Office européen des brevets

(11) EP 4 556 456 A1

(12) EUROPEAN PATENT APPLICATION

(43) Date of publication:
**21.05.2025 Bulletin 2025/21**

(21) Application number: **23206933.6**

(22) Date of filing: **31.10.2023**

(51) International Patent Classification (IPC):
***C07C 29/10*** *(2006.01)*

(52) Cooperative Patent Classification (CPC):
(C-Sets available)
**C07C 29/1518; C07C 1/20; C07C 29/106; C07C 41/03; C07D 301/04; C08G 65/08; C25B 1/04**
(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **BASF SE**
**67056 Ludwigshafen am Rhein (DE)**

(72) Inventors:
- **DIETL, Harald**
**67056 Ludwigshafen am Rhein (DE)**
- **HUEFFER, Stephan**
**67056 Ludwigshafen am Rhein (DE)**
- **WEISS, Thomas**
**67056 Ludwigshafen am Rhein (DE)**
- **KRUEGER, Marco**
**67056 Ludwigshafen am Rhein (DE)**
- **GUMLICH, Kai**
**67056 Ludwigshafen am Rhein (DE)**

(74) Representative: **BASF IP Association**
**BASF SE**
**GBI - Z078**
**67056 Ludwigshafen (DE)**

# (54) PROCESS FOR MAKING ETHYLENE GLYCOLS AND ETHOXYLATES BASED ON NON-FOSSIL ENERGY

(57) The present invention relates to a process for making ethylene glycols, selected from mono ethylene glycol, oligo ethylene glycols, poly ethylene glycols, and mixtures thereof, and alkanol ethoxylates, based on non-fossil energy, ethylene glycols, selected from mono ethylene glycol, oligo ethylene glycols, poly ethylene glycols, and mixtures thereof, and alkanol ethoxylates, having a low molar share of deuterium, the use of the molar share of deuterium in hydrogen and downstream compounds based on hydrogen for tracing the origin, especially the energetic origin, of the hydrogen and downstream compounds based on hydrogen, wherein the compounds are ethylene glycols, selected from mono ethylene glycol, oligo ethylene glycols, poly ethylene glycols, and mixtures thereof, and alkanol ethoxylates, a process for tracing the origin, especially the energetic origin, of hydrogen and downstream compounds based on hydrogen by determining the molar share of deuterium in hydrogen and said downstream compounds based on hydrogen, wherein the compounds are ethylene glycols, selected from mono ethylene glycol, oligo ethylene glycols, poly ethylene glycols, and mixtures thereof, and alkanol ethoxylates, coolants, comprising such mono ethylene glycol, brake fluids comprising such oligo ethylene glycols and/or such alkanol ethoxylates, cosmetics, shampoos, or nonionic or ionic detergents comprising such poly ethylene glycols and/or such alkanol ethoxylates, poly ethylene terephthalate, comprising such mono ethylene glycol.

EP 4 556 456 A1

(52) Cooperative Patent Classification (CPC): (Cont.)

C-Sets
**C07C 1/20, C07C 11/04;**
**C07C 29/106, C07C 31/202;**
**C07C 29/1518, C07C 31/04;**
**C07C 41/03, C07C 43/11**

## Description

The present invention relates to a process for making ethylene glycols, selected from mono ethylene glycol, oligo ethylene glycols, poly ethylene glycols, and mixtures thereof, and alkanol ethoxylates, based on non-fossil energy, ethylene glycols, selected from mono ethylene glycol, oligo ethylene glycols, poly ethylene glycols, and mixtures thereof, and alkanol ethoxylates, having a low molar share of deuterium, the use of the molar share of deuterium in hydrogen and downstream compounds based on hydrogen for tracing the origin, especially the energetic origin, of the hydrogen and downstream compounds based on hydrogen, wherein the compounds are ethylene glycols, selected from mono ethylene glycol, oligo ethylene glycols, poly ethylene glycols, and mixtures thereof, and alkanol ethoxylates, a process for tracing the origin, especially the energetic origin, of hydrogen and downstream compounds based on hydrogen by determining the molar share of deuterium in hydrogen and said downstream compounds based on hydrogen, wherein the compounds are ethylene glycols, selected from mono ethylene glycol, oligo ethylene glycols, poly ethylene glycols, and mixtures thereof, and alkanol ethoxylates, coolants, comprising such mono ethylene glycol, brake fluids comprising such oligo ethylene glycols and/or such alkanol ethoxylates, cosmetics, shampoos, or nonionic or ionic detergents comprising such poly ethylene glycols and/or such alkanol ethoxylates, polyesters or poly ethylene terephthalate, comprising such mono ethylene glycol.

Ethylene glycols are colorless, viscous liquids that are produced by the reaction between water and ethylene oxide (EO). Identified many years ago, ethylene glycols are a key ingredient in a number of important product formulations such as cosmetics, shampoos, soaps or detergents and personal hygiene applications, coolants, brake fluids, and polymers, such as poly ethylene terephthalate. They can also be used in the production of nonionic detergents, emulsifiers, and soaps, as well as in emulsion paints, polishes, and cleansers. There are 3 types of ethylene glycols: mono ethylene glycol, oligo ethylene glycols, poly ethylene glycols. The formation of mono ethylene glycol, oligo ethylene glycols, poly ethylene glycols depends on the stoichiometry between water and ethylene oxide molecules in the reaction.

According to the present application ethylene glycols are of the general formula

(I) $HO\text{-}[\text{-}CH_2\text{-}CH_2\text{-}O\text{-}]_m\text{-}H$

wherein

m is a positive integer from 1 to 100.

In the context of the present application

- for mono ethylene glycol is m = 1,
- for oligo ethylene glycols m is from 2 to 4, and
- for poly ethylene glycols m is from 5 to 100.

Alkanol ethoxylates are obtainable by reaction of alkanols with ethylene oxide and are key ingredient in a number of important product formulations such as cosmetics, shampoos, soaps or detergents and personal hygiene applications, brake fluids. The degree of ethoxylation depends on the stoichiometry between alkanol and ethylene oxide molecules in the reaction.

According to the present application alkanol ethoxylates are of the general formula

(II) $H_{2n+1}C_n\text{-}O\text{-}[\text{-}CH_2\text{-}CH_2\text{-}O\text{-}]_m\text{-}H$

wherein

n is a positive integer from 1 to 20, and
m is a positive integer from 1 to 100.

It is clear that the reaction mixtures of ethylene glycols and alkanol ethoxylates are mixtures of a polymerisation of ethylene oxide and, therefore, exhibit a molecular weight distribution. Therefore, while for each polymer chain m is an integer, for the statistic reaction mixture m may be a rational number.

Ethylene oxide is prepared from ethylene which at present is predominantly produced from fossil sources, usually from naphtha in a steam cracking process.

However, the petrochemical steam cracking process has its negative impacts with regard to its carbon footprint including the consumption of a lot of fossil-based natural resources and energy and is therefore less preferred.

It is therefore an object of the present invention to provide environmentally friendly ethylene glycols, selected from mono ethylene glycol, oligo ethylene glycols, poly ethylene glycols, and mixtures thereof, and alkanol ethoxylates, and an environmentally friendly process for making the same, that process uses as little fossil-based energy as possible.

The object is achieved by a process for making ethylene glycols, selected from mono ethylene glycol, oligo ethylene glycols, poly ethylene glycols, and mixtures thereof, and alkanol ethoxylates, wherein said process comprises the following steps:

(a) providing hydrogen with a molar share of deuterium ≤ 100 ppm, preferably in the range of from 10 to ≤ 95 ppm, more preferably in the range of from 10 to ≤ 90 ppm, most preferably in the range of from 10 to ≤ 80 ppm, based on the total hydrogen content, by electrolysis based on electrical power generated at least in part from non-fossil energy,
(b) providing carbon oxides, preferably carbon dioxide,
(c) reacting the hydrogen from step (a) with carbon oxides, preferably carbon dioxide from step (b) to form methanol,
(d) converting the methanol from step (c) to ethylene and further to ethylene oxide,
(e) converting water resp. alkanols $C_nH_{2n+1}$-OH with ethylene oxide from step (d) to ethylene glycols resp. alkanol ethoxylates in one or more steps.

In a further embodiment of the present invention, the object is achieved by ethylene glycols of formula (I), selected from mono ethylene glycol, oligo ethylene glycols, poly ethylene glycols and mixtures thereof, wherein the molar share of deuterium is

not more than

$$[(2 \times 150) + (4 \times m \times 100)] / (2 + (4 \times m)) \text{ ppm},$$

preferably in the range of from

$$[(2 \times 150) + (4 \times m \times 10)] / (2 + (4 \times m))$$

to

$$[(2 \times 150) + (4 \times m \times 98)] / (2 + (4 \times m)) \text{ ppm},$$

more preferably from

$$[(2 \times 150) + (4 \times m \times 10)] / (2 + (4 \times m))$$

to

$$[(2 \times 150) + (4 \times m \times 95)] / (2 + (4 \times m)) \text{ ppm},$$

most preferably from

$$[(2 \times 150) + (4 \times m \times 10)] / (2 + (4 \times m))$$

to

$$[(2 \times 150) + (4 \times m \times 90)] / (2 + (4 \times m)) \text{ ppm},$$

based on the total hydrogen content.

In a further embodiment of the present invention, the object is achieved by alkanol ethoxylates of formula (II) and mixtures thereof, wherein the molar share of deuterium is

not more than

$$[(((2 \times n) + 2) \times 150) + (4 \times m \times 100)] / (((2 \times n) + 2) + (4 \times m)) \text{ ppm},$$

preferably in the range of
from

$$[(((2 \times n) + 2) \times 150) + (4 \times m \times 10)] / (((2 \times n) + 2) + (4 \times m))$$

to

$$[(((2 \times n) + 2) \times 150) + (4 \times m \times 98)] / (((2 \times n) + 2) + (4 \times m)) \text{ ppm},$$

more preferably in the range of
from

$$[(((2 \times n) + 2) \times 150) + (4 \times m \times 10)] / (((2 \times n) + 2) + (4 \times m))$$

to

$$[(((2 \times n) + 2) \times 150) + (4 \times m \times 95)] / (((2 \times n) + 2) + (4 \times m)) \text{ ppm}$$

most preferably in the range of
from

$$[(((2 \times n) + 2) \times 150) + (4 \times m \times 10)] / (((2 \times n) + 2) + (4 \times m))$$

to

$$[(((2 \times n) + 2) \times 150) + (4 \times m \times 90)] / (((2 \times n) + 2) + (4 \times m)) \text{ ppm},$$

based on the total hydrogen content.

Further, it is important that the origin of the hydrogen and downstream compounds obtained by clean energy can be tracked in a reliable way.

This is especially important in order to ensure that:

- Hydrogen and downstream compounds have been produced in accordance with sustainability criteria (trustworthy certificates for customers and final customers).
- Renewable attributes aren't subject to double counting.

Companies are placing increasing importance on sourcing green energy. Because of this, tracking systems have to be developed for the origin of the energy used in the preparation of hydrogen and downstream compounds.

This object is also achieved by use of the molar share of deuterium in hydrogen and downstream compounds based on hydrogen for tracing the origin, especially the energetic origin, of the hydrogen and downstream compounds based on hydrogen, wherein the compounds are ethylene glycols, selected from mono ethylene glycol, oligo ethylene glycols, poly ethylene glycols, and mixtures thereof, and alkanol ethoxylates, and a process for tracing the origin, especially the energetic origin, of hydrogen and downstream compounds based on hydrogen by determining the molar share of deuterium in hydrogen and said downstream compounds based on hydrogen, wherein the compounds are ethylene glycols, selected from mono ethylene glycol, oligo ethylene glycols, poly ethylene glycols, and mixtures thereof, and alkanol ethoxylates. Methods for determination of the molar share of deuterium in hydrogen and downstream compounds based on hydrogen are known to a person skilled in the art. A suitable method is described in the examples of the present application.

A further environmental benefit of the environmentally friendly ethylene glycols, selected from mono ethylene glycol, oligo ethylene glycols, poly ethylene glycols, and mixtures thereof, and alkanol ethoxylates, according to the present invention is their use in coolants, brake fluids, poly ethylene terephthalate, cosmetics, shampoos, or nonionic or ionic detergents according to the present invention. The ethylene glycols, selected from mono ethylene glycol, oligo

ethylene glycols, poly ethylene glycols, and mixtures thereof, and alkanol ethoxylates are produced using as little fossil-based energy as possible, ideally no fossil-based energy, and do therefore only add as little as possible, ideally nothing, to $CO_2$ emission.

The molar share of deuterium in hydrogen and downstream compounds based on hydrogen is given in the present application in ppm, based on the total hydrogen content, which is the mol-ppm content of deuterium, based on the total hydrogen content (in hydrogen or in the compounds discussed, respectively).

The deuterium content of hydrogen and downstream compounds based on hydrogen is given in the present application in atom-ppm based on the total molar hydrogen content (total atoms of protium $^{1}H$ and deuterium $^{2}H$). The terms "deuterium content" and "molar share of deuterium" are used synonymously throughout the application.

In physical organic chemistry, a kinetic isotope effect is the change in the reaction rate of a chemical reaction when one of the atoms in the reactants is replaced by one of its isotopes. Formally, it is the ratio of rate constants $k_L$ / $k_H$ for the reactions involving the light ($k_L$) and the heavy ($k_H$) isotopically substituted reactants (isotopologues). This change in reaction rate is a quantum mechanical effect that primarily results from heavier isotopologues having lower vibrational frequencies compared to their lighter counterparts. In most cases, this implies a greater energetic input needed for heavier isotopologues to reach the transition state, and consequently, a slower reaction rate.

Isotopic rate changes are most pronounced when the relative mass change is greatest, since the effect is related to vibrational frequencies of the affected bonds. For instance, changing a hydrogen atom (H) to its isotope deuterium (D) represents a 100 % increase in mass, whereas in replacing $^{12}C$ with $^{13}C$, the mass increases by only 8 percent. The rate of a reaction involving a C-H bond is typically 6-10 times faster than the corresponding C-D bond, whereas a $^{12}C$ reaction is only 4 percent faster than the corresponding $^{13}C$ reaction.

A primary kinetic isotope effect may be found when a bond to the isotope atom is being formed or broken. A secondary kinetic isotope effect is observed when no bond to the isotope atom in the reactant is broken or formed. Secondary kinetic isotope effects tend to be much smaller than primary kinetic isotope effects; however, secondary deuterium isotope effects can be as large as 1.4 per deuterium atom.

Process comprising steps (a) to (e) as mentioned above:

Step (a)

Step (a) concerns the provision of hydrogen with a molar share of deuterium below 90 ppm, based on the total hydrogen content, by electrolysis based on electrical power generated at least in part from non-fossil energy.

The electrical power is generated at least in part from non-fossil resources.

The term "at least in part" means that part of the electrical power can still be produced from fossil fuels, preferably from natural gas, since combustion of natural gas causes much lower carbon dioxide emission per Megajoule of electrical energy produced than combustion of coal. However, the portion of electrical energy produced from fossil fuels should be as low as possible, preferably ≤ 50%, preferably ≤ 30%, most preferably ≤ 20%, further most preferably ≤ 10%. In one embodiment, the electrical power is generated exclusively from non-fossil resources. Various methods for certification and tracking of the "energy source mix" have been set up based on local legislations. Certificates such as "Non-Fossil Certificate Contracts" are common practice for tracking the ratio of non-fossil energy used in industrial processes and related products (https://www.ekoenergy.org/ecolabel/criteria/tracking/)

Preferably, the electrical power is generated at least in part from wind power, solar energy (thermal, photovoltaic and concentrated solar power), hydroelectricity (tidal power, wave power, hydroelectric dams, In-river-hydrokinetics), geothermal energy, ambient or industrial heat captured by heat pumps, bioenergy (biofuel, biomass), the renewable part of waste energy sources or nuclear energy (fission, fusion).

In a further embodiment, the electrical power is generated at least in part from renewable resources, preferably from wind power, solar energy (thermal, photovoltaic and concentrated solar power), hydroelectricity (tidal power, wave power, hydroelectric dams, In-river-hydrokinetics), geothermal energy, ambient heat captured by heat pumps, bioenergy (biofuel, biomass), or the renewable part of waste.

The types of electrical power resources mentioned above are generally known by a person skilled in the art.

In one preferred embodiment of the inventive process, the electrical power from nonfossil resources used in the electrolysis according to the invention can be generated at least in part by nuclear energy. The nuclear energy can be obtained by fission or fusion.

Fission occurs when a neutron enters a larger atomic nucleus, forcing it to excite and spilt into two smaller atoms- also known as fission products. Additional neutrons are also released that can initiate a chain reaction. When each atom splits, a tremendous amount of energy is released. Uranium and plutonium isotopes are most commonly used for fission reactions in nuclear power reactors because they are easy to initiate and control. The energy released by fission in these reactors heats water into steam. The steam is used to spin a turbine to produce carbon-free electricity.

Fusion occurs when two light nuclei are combined to form a heavier atom, like when two hydrogen isotopes (e.g. deuterium and tritium) fuse to form one helium atom. This is the same process that powers the sun and creates huge

amounts of energy-several times greater specific energy per nucleus than fission. It also doesn't produce highly radioactive fission products. Fusion reactors are already operating in pilot scale and scale-up to commercial scale is targeted by various entities.

In one preferred embodiment of the inventive process, the electrical power used in electrolysis is generated at least in part from wind power. Wind power can be used to run wind turbines. Modern utility-scale wind turbines range from around 600 kW to 9 MW of rated power. The power available from the wind is a function of the cube of the wind speed, so as wind speed increases, power output increases up to the maximum output for the particular turbine. Areas where winds are stronger and more constant, such as offshore and high-altitude sites, are preferred locations for wind farms.

In one further preferred embodiment of the inventive process, the electrical power used in electrolysis is generated at least in part from solar power, particularly preferred from photovoltaic systems. A photovoltaic system converts light into electrical direct current (DC) by taking advantage of the photoelectric effect. Concentrated solar power (CSP) systems use lenses or mirrors and tracking systems to focus a large area of sunlight into a small beam. CSP-Stirling currently has by far the highest efficiency among all solar energy technologies.

In one preferred embodiment of the inventive process, the electrical power used in electrolysis is generated at least in part from hydropower. There are many forms of hydropower. Traditionally, hydroelectric power comes from constructing large hydroelectric dams and reservoirs. Small hydro systems are hydroelectric power installations that typically produce up to 50 MW of power. They are often used on small rivers or as a low-impact development on larger rivers. Run-of-the-river hydroelectricity plants derive energy from rivers without the creation of a large reservoir. The water is typically conveyed along the side of the river valley (using channels, pipes and/or tunnels) until it is high above the valley floor, whereupon it can be allowed to fall through a penstock to drive a turbine.

Wave power, which captures the energy of ocean surface waves, and tidal power, converting the energy of tides, are two forms of hydropower with future potential.

In one further preferred embodiment of the inventive process, the electrical power used in electrolysis is generated at least in part from geothermal energy. Geothermal energy is the heat that comes from the sub-surface of the earth. It is contained in the rocks and fluids beneath the earth's crust and can be found as far down to the earth's hot molten rock, magma.

To produce power from geothermal energy, wells are dug a mile deep into underground reservoirs to access the steam and hot water there, which can then be used to drive turbines connected to electricity generators. There are three types of geothermal power plants; dry steam, flash and binary.

Dry steam is the oldest form of geothermal technology and takes steam out of the ground and uses it to directly drive a turbine. Flash plants use high-pressure hot water into cool, low-pressure water whilst binary plants pass hot water through a secondary liquid with a lower boiling point, which turns to vapor to drive the turbine.

It is also possible to pump water down to a geothermally active layer and use the thus heated water or steam for energy production.

In one further preferred embodiment of the inventive process, the electrical power used in electrolysis is generated at least in part from biomass. Biomass is biological material derived from living, or recently living organisms. It most often refers to plants or plant-derived materials which are specifically called lignocellulosic biomass. As an energy source, biomass can either be used directly via combustion to produce heat (e.g. heat from fermentation processes) or electricity, or indirectly after converting it to various forms of biofuel and gas. Conversion of biomass to biofuel can be achieved by different methods which are broadly classified into: thermal, chemical, and biochemical methods. Wood was the largest biomass energy source as of 2012; examples include forest residues - such as dead trees, branches and tree stumps -, yard clippings, wood chips and even municipal solid waste. Industrial biomass can be grown from numerous types of plants, including miscanthus, switchgrass, hemp, corn, poplar, willow, sorghum, sugarcane, bamboo, and a variety of tree species, ranging from eucalyptus to oil palm (palm oil).

Plant energy is produced by crops specifically grown for use as fuel that offer high biomass output per hectare with low input energy. The grain can be used for liquid transportation fuels while the straw can be burned to produce heat or electricity. Biomass can be converted to other usable forms of energy such as methane gas or transportation fuels such as ethanol and biodiesel.

Rotting garbage, and agricultural and human waste, all release methane gas - also called landfill gas or biogas. Crops, such as corn and sugarcane, can be fermented to produce the transportation fuel, ethanol. Biodiesel, another transportation fuel, can be produced from left-over food products such as vegetable oils and animal fats.

Biopower technologies convert renewable biomass fuels into heat and electricity using processes like those used with fossil fuels. There are three ways to harvest the energy stored in biomass to produce biopower: burning, bacterial decay, and conversion to a gas or liquid fuel. Biopower can offset the need for carbon fuels burned in power plants, thus lowering the carbon intensity of electricity generation. Unlike some forms of intermittent renewable energy, biopower can increase the flexibility of electricity generation and enhance the reliability of the electric grid.

The electrolysis in step (a) is generally an electrolysis of water.

Electrolysis of water is an environmentally friendly method for production of hydrogen because it uses renewable

$H_2O$ and produces only pure oxygen as by-product.

According to the present invention, the electrolysis which is generally a water electrolysis utilizes as electrical power direct current (DC) at least in part from non-fossil energy resources.

It is now observed as a key observation of the present application that by the electrolysis of water, the deuterium atom content of the hydrogen is lower than in the hydrogen generated petrochemically, for example as contained in fossil-based synthesis gas, i.e. ≤ 100 ppm, preferably in the range of from 10 to ≤ 95 ppm, more preferably in the range of from 10 to ≤ 90 ppm, most preferably in the range of from 10 to ≤ 80 ppm, based on the total hydrogen content. The deuterium atom content in electrolytically produced hydrogen may be as low as 10 ppm. The remaining deuterium is mainly present in the form of D-H rather than $D_2$.

One suitable water electrolysis process is alkaline water electrolysis. Hydrogen production by alkaline water electrolysis is a well-established technology up to the megawatt range for a commercial level. In alkaline water electrolysis initially at the cathode side two water molecules of alkaline solution (KOH/NaOH) are reduced to one molecule of hydrogen ($H_2$) and two hydroxyl ions ($OH^-$). The produced $H_2$ emanates from the cathode surface in gaseous form and the hydroxyl ions ($OH^-$) migrate under the influence of the electrical field between anode and cathode through the porous diaphragm to the anode, where they are discharged to half a molecule of oxygen ($O_2$) and one molecule of water ($H_2O$). Alkaline electrolysis operates at lower temperatures such as 30-100°C with alkaline aqueous solution (KOH/NaOH) as the electrolyte, the concentration of the electrolyte being about 20% to 30 %. The diaphragm in the center of the electrolysis cell separates the cathode and anode and also separates the produced gases from their respective electrodes, avoiding the mixing of the produced gases.

An overview of hydrogen production by alkaline water electrolysis powered by renewable energy is given in J. Brauns and T. Turek in Processes, 8(2) (2020), pp. 248.

In one further embodiment of the inventive process, hydrogen is provided by polymer electrolyte membrane water electrolysis. Variants of polymer electrolyte membrane water electrolysis are proton exchange membrane water electrolysis (PEMWE, PEM water electrolysis) and anion exchange membrane water electrolysis (AEMWE, AEM water electrolysis).

PEM water electrolysis technology is similar to the PEM fuel cell technology, where solid poly-sulfonated membranes (Nafion®), fumapem®) are used as an electrolyte (proton conductor). These proton exchange membranes have many advantages such as low gas permeability, high proton conductivity ($0.1 \pm 0.02$ S $cm^{-1}$), low thickness (20-300 μm), and allow high-pressure operation. In terms of sustainability and environmental impact, PEM water electrolysis is one of the most favorable methods for conversion of renewable energy to highly pure hydrogen. PEM water electrolysis has great advantages such as compact design, high current density (above 2 A $cm^{-2}$), high efficiency, fast response, operation at low temperatures (20-90°C) and production of ultrapure hydrogen. The state-of-the-art electrocatalysts for PEM water electrolysis are highly active noble metals such as Pt/Pd for the hydrogen evolution reaction (HER) at the cathode and $IrO_2/RuO_2$ for the oxygen evolution reaction (OER) at the anode.

One of the largest advantages of PEM water electrolysis is its ability to operate at high current densities. This can result in reduced operational costs, especially for systems coupled with very dynamic energy sources such as wind and solar power, where sudden spikes in energy output would otherwise result in uncaptured energy. The polymer electrolyte allows the PEM water electrolyzer to operate with a very thin membrane (ca. 100-200 μm) while still allowing high operation pressure, resulting in low ohmic losses, primarily caused by the conduction of protons across the membrane (0.1 S/cm), and a compressed hydrogen output.

The PEM water electrolyzer utilizes a solid polymer electrolyte (SPE) to conduct protons from the anode to the cathode while insulating the electrodes electrically. Under standard conditions the enthalpy required for the formation of water is 285.9 kJ/mol. One portion of the required energy for a sustained electrolysis reaction is supplied by thermal energy and the remainder is supplied through electrical energy.

The half reaction taking place on the anode side of a PEM water electrolyzer is commonly referred to as the Oxygen Evolution Reaction (OER). Here the liquid water reactant is supplied to a catalyst where it is oxidized to oxygen, protons and electrons.

The half reaction taking place on the cathode side of a PEM water electrolyzer is commonly referred to as the Hydrogen Evolution Reaction (HER). Here the protons that have moved through the membrane are reduced to gaseous hydrogen.

PEMs can be made from either pure polymer membranes or from composite membranes, where other materials are embedded in a polymer matrix. One of the most common and commercially available PEM materials is the fluoropolymer PFSA (e.g. Nafion®, a DuPont product).

While Nafion® is an ionomer with a perfluorinated backbone like Teflon, there are many other structural motifs used to make ionomers for proton-exchange membranes. Many use polyaromatic polymers, while others use partially fluorinated polymers.

An overview over hydrogen production by PEM water electrolysis is given in S. Kumar and V. Himabindu, Material Science for Energy Technologies 2 (2019), pp. 4442 - 4454.

The AEM water electrolysis technology adopts low-cost catalytic materials, as in alkaline electrolysis, and a solid polymer electrolyte architecture, as in PEM electrolysis technology. AEM electrolysis technology operates in an alkaline environment (pH ~ 10), making it possible the use modest non-noble-metal electrocatalysts (i.e. platin group metal free catalysts = PGM free catalysts), whilst accommodating a zero-gap architecture. The membrane used in this type of electrolysis is a polymeric membrane, containing quaternary ammonium salts. It is relatively inexpensive and has low interaction with atmospheric $CO_2$.

Catalysts:

As examples for hydrogen evolution reaction (HER) catalysts, catalysts based on Ni-Mo alloyed materials are suitable.

As examples for oxygen evolution reaction (OER) catalysts, high activity of transition metal mixed oxides are suitable. Specific examples are CuxCo3_xO4, NiCo2O4:Fe and Ni-Fe alloys on Ni foam supports, for example the PGM-free catalysts (Ni-Fe, Ni-Mo, Ni/(CeO2-La2O3)/C and CuxCo3_xO4).

Membranes and ionomers:

The chemical stability of AEMs under alkaline conditions has improved markedly due to the development of stabilized functional groups on the polymer backbone. This allows the use of such membranes in AEM electrolysis at higher temperatures for

long periods. Suitable membranes and ionomers are known by a person skilled in the art and for example described in the review mentioned below. One example is the commercial membrane Tokuyama A201.

Membrane electrode assembly preparation and cell performance:

The physical and electrochemical characterization of the membrane electrode assembly prepared by either the catalyst-coated substrate (CCS) or the catalyst-coated membrane (CCM) method suggests that the CCM is preferable because improvements in ionic conductivity far outweigh any improvements in electronic conductivity.

Liquid electrolyte: Pure water feeds generally result in poor current densities while 1%

$K_2CO_3$ or dilute KOH solutions give good results. A good electrolysis performance is achieved with a 1% $K_2CO_3$ electrolyte. It is therefore preferable that the water electrolyte comprises 0.1 to 2 wt% $K_2CO_3$ or KOH.

An overview over hydrogen production by anion exchange membrane water electrolysis is given in H. A. Miller et al., Sustainable Energy Fuels, 2020, 4, pp. 2114 - 2133.

Beside the alkaline water electrolysis, the AEM and PEM, a further commercially available electrolysis technology is the solid oxide electrolysis (SOE).

SOEC (solid oxide electrolysis cell) feeds water into the cathode and the water undergoes water reduction reaction (WRR), which converts water into hydrogen gas and oxide ions. This hydrogen gas is later brought to purification modules to separate hydrogen gas from the remaining water. Then, the oxide ions migrate from cathode to anode and they release electrons to external circuit to become oxygen gas *via* oxygen evolution reaction (OER). Typically, the operating temperatures for SOFCs are from 800 to 1,000 °C, because high temperatures are required to thermally activate the migration of oxide ions and to facilitate electrochemical reactions on both electrodes. As a result, the overall efficiency is improved. The SOEC is for example described in K. Kamlungsua et al., FUEL CELLS 20, 2020, No. 6, 644-649.

Preferably, the electrolysis in step (a) is a water electrolysis, more preferably PEM water electrolysis, alkaline water electrolysis, or AEM water electrolysis.

In a further preferred embodiment, the electrolysis in step (a) is a solid oxide water electrolysis (SOE).

It is known in the art that deuterium in the evolving hydrogen gas can be depleted with regard to feed water in water electrolysis, e.g. polymer electrolyte membrane water electrolysis. The depletion factor is depending on the electrolysis conditions (water flow, current density). Since the average deuterium content (molar share of deuterium) of water is about 150 ppm, based on the total hydrogen content, hydrogen provided in step (a) of the inventive process has a molar share of deuterium (deuterium content) of ≤ 100 ppm, preferably in the range of from 10 to ≤ 95 ppm, more preferably in the range of from 10 to ≤ 90 ppm, most preferably in the range of from 10 to ≤ 80 ppm, based on the total hydrogen content, or even lower.

Generally, any water source can be used in the preferred water electrolysis in step (a). However, since the hydrogen prepared in step (a) has a molar share of deuterium (deuterium content) below ≤ 100 ppm, preferably in the range of from 10 to ≤ 95 ppm, more preferably in the range of from 10 to ≤ 90 ppm, most preferably in the range of from 10 to ≤ 80 ppm, based on the total hydrogen content, it is preferable to use water having a molar share of deuterium (deuterium content) below 160 ppm, based on the total hydrogen content.

Vienna Standard Mean Ocean Water (VSMOW) is an isotopic water standard defined in 1968 by the International

Atomic Energy Agency. Despite the somewhat misleading phrase "ocean water", VSMOW refers to pure water ($H_2O$) and does not include any salt or other substances usually found in seawater. VSMOW serves as a reference standard for comparing hydrogen and oxygen isotope ratios, mostly in water samples. Very pure, distilled VSMOW water is also used for making high accuracy measurement of water's physical properties and for defining laboratory standards since it is considered to be representative of "average ocean water", in effect representing the water content of Earth.

The isotopic composition of VSMOW water is specified as ratios of the molar abundance of the rare isotope in question divided by that of its most common isotope and is expressed as parts per million (ppm). For instance $^{16}O$ (the most common isotope of oxygen with eight protons and eight neutrons) is roughly 2,632 times more prevalent in sea water than is $^{17}O$ (with an additional neutron). The isotopic ratios of VSMOW water are defined as follows:

$^{2}H$ / $^{1}H$ = 155.76 $\pm$0.1 ppm (a ratio of 1 part per approximately 6420 parts)
$^{3}H$ / $^{1}H$ = 1.85 $\pm$0.36 × $10^{-11}$ ppm (a ratio of 1 part per approximately 5.41 × $10^{16}$ parts, ignored for physical properties-related work)
$^{18}O$ / $^{16}O$ = 2005.20 $\pm$0.43 ppm (a ratio of 1 part per approximately 498.7 parts)
$^{17}O$ / $^{16}O$ = 379.9 $\pm$1.6 ppm (a ratio of 1 part per approximately 2632 parts)
(see: https://en-academic.com/dic.nsf/enwiki/753132)

More preferably, the water in step (a) has an average deuterium content of 1 ppm (super light water) to 156 ppm, based on the total hydrogen content, most preferably 2 ppm to 150 ppm, based on the total hydrogen content.

Processes for the depletion of deuterium in water are known by a person skilled in the art. However, said processes are generally energy consuming electrolysis processes as e.g. described in CN103848399A.

In the case that deuterium depleted water is used, it is therefore preferred to employ deuterium depleted water obtained from the following resources:

- A byproduct of "heavy water" ($D_2O$) production (heavy water has applications in organic chemistry, drug development, and nuclear reactors); (deuterium content about 10-120 ppm)
- High mountain water; (deuterium content about 120-150 ppm)
- Surface river and lake water; (deuterium content about 130-150 ppm)
- Any water source with seasonally low deuterium content e.g. water collected at low temperature (cold winter water contains less deuterium than warm summer water); e.g. water obtained in winter time, e.g. from snow or ice; (deuterium content about 120-150 ppm)
- Pole (arctic) water and antarctic glacier water (deuterium content about 90-150 ppm)
- Low salinity sea water e.g. close to river mouths, desalinated sea water or brackish water and waste water treatment effluent water; (deuterium content about 130 - 155 ppm)

Step (b)

Step (b) concerns providing carbon oxides, preferably carbon dioxide.

The carbon dioxide ($CO_2$) and/or carbon monoxide (CO) used may originate from any source, such as exhaust gases, combustion gases, fermentation gases, synthesis gas, or separation from air (also known as carbon capture (CC) or carbon capture and utilisation (CCU)).

An advantage in the carbon footprint arises if the carbon dioxide ($CO_2$) and/or carbon monoxide (CO) used in the process is not produced from fossil sources specifically for the process. On the other hand, the use of carbon dioxide ($CO_2$) and/or carbon monoxide (CO) from exhaust gases or combustion gases, which would otherwise be released into the atmosphere without their use in any process, is advantageous.

Preferably, the carbon dioxide ($CO_2$) and/or carbon monoxide (CO) used in the process comes from renewable raw materials, for example from combustion processes or fermentation.

It is also advantageous if carbon dioxide ($CO_2$) is separated from the air and used in the process (carbon capture and utilisation (CCU)). In this case, the monoethylene glycols or alkanol ethoxylates obtained from the process represent a carbon sink.

In preferred embodiments, the carbon dioxide that is provided in step (b) is captured from industrial flue gases or from ambient air. All available capture technologies may be used.

An overview of commercial $CO_2$ capturing technologies is given in Koytsoumoa et al., The Journal of Supercritical Fluids, Volume 132, February 2018, Pages 3-16.

Capturing $CO_2$ is most cost-effective at point sources, such as large carbon-based energy facilities, industries with major $CO_2$ emissions (e.g. cement production, ammonia synthesis, steelmaking, fertilizer plants), natural gas processing, synthetic fuel plants and fossil fuel-based hydrogen production plants. Extracting $CO_2$ from air is possible, although the lower concentration of $CO_2$ in air compared to combustion sources complicates the engineering and makes

the process therefore more expensive.

In some preferred embodiments, the carbon dioxide that is provided in step (b) is captured from industrial flue gases.

The main industrial sources of $CO_2$ are power plants based on burning of fossil fuels, oil refineries, biogas sweetening (e.g. fermentation) as well as the production of chemicals. Relevant chemical production processes are e.g. naphta cracking for $C_1$-$C_4$ olefins and $C_6$ aromatics as well as downstream chemicals such as especially ammonia and other $CO_2$-intensive products). Furthermore industrial paper, food, cement, mineral and iron and steel production can be named as examples.

In post combustion capture, the $CO_2$ is removed after combustion of the fossil fuel-this is the scheme that would apply to fossil-fuel power plants. $CO_2$ is captured from flue gases at power stations or other point sources. Absorption, or carbon scrubbing with amines is the dominant capture technology. It is the only carbon capture technology so far that has been used industrially. Suitable post carbon capture methods are for example absorption (chemical, physical), adsorption (chemical, physical), membrane processes, biological and cryogenic processes.

Pre-conversion capture means capturing $CO_2$ generated as an undesired co-product of an intermediate reaction of a conversion process. Some examples include the production of ammonia and coal gasification in power plants. In ammonia production, $CO_2$ that is co-produced with hydrogen during steam reforming must be removed before the ammonia synthesis can take place - absorption in monoethanolamine (MEA) and/or diethanolamine (DEA) is commonly used for these purposes. Similarly, in an integrated gasification combined cycle (IGCC) power plant, $CO_2$ must be separated from hydrogen. This is typically achieved using physical solvents such as Selexol™ and Rectisol™. Note that, when applied in power plants, pre-conversion capture is also referred to as pre-combustion capture.

Oxy-fuel combustion technology involves the combustion of carbonaceous fuel in a stream of pure oxygen instead of air. Since the oxidant ($O_2$) is free of other components in the air (such as nitrogen), the $CO_2$ concentration in the flue gas will be very high, while the water vapor content can be easily removed.

$CO_2$ adsorbs to a MOF (Metal-organic framework) through physisorption or chemisorption based on the porosity and selectivity of the MOF leaving behind a $CO_2$ poor gas stream. The $CO_2$ is then stripped off the MOF using temperature swing adsorption (TSA) or pressure swing adsorption (PSA) so the MOF can be reused.

In some other preferred embodiments, the carbon dioxide that is provided in step (b) is captured from ambient air.

Direct air capture (DAC) is a process of capturing carbon dioxide ($CO_2$) directly from the ambient air and generating a concentrated stream of $CO_2$ for sequestration or utilization or production of carbon-neutral fuel. Carbon dioxide removal is achieved when ambient air makes contact with chemical media, typically an aqueous alkaline solvent or sorbents. These chemical media are subsequently stripped of $CO_2$ through the application of energy (namely heat), resulting in a $CO_2$ stream that can undergo dehydration and compression, while simultaneously regenerating the chemical media for reuse.

In Chen, Lackner et al., Angew. Chem. Int. Ed. 2020, 59, 6984 - 7006, "Sorbents for the Direct Capture of CO2 from Ambient Air" describes major types of sorbents designed to capture CO2 from ambient air categorized by the sorption mechanism: physisorption, chemisorption, and moisture-swing sorption.

In Kommalapati et al., Energy Technol. 2017, 5, 822 - 833, polyethylenimine applications in carbon dioxide capture and separation are described.

Dilute $CO_2$ can be efficiently separated using an anionic exchange polymer resin called Marathon MSA, which absorbs air $CO_2$ when dry, and releases it when exposed to moisture. A large part of the energy for the process is supplied by the latent heat of phase change of water. Other substances which can be used are metal-organic frameworks (or MOF's). Membrane separation of $CO_2$ rely on semi-permeable membranes.

Suitable carbon capturing processes are mentioned above and known in the art.

Step (c)

Step (c) concerns reacting the hydrogen from step (a) with carbon oxides, preferably carbon dioxide to form methanol.

Suitable carbon oxides are carbon monoxide, carbon dioxide or mixtures of both, wherein carbon dioxide is preferred.

Process conditions for the hydrogenation of carbon monoxide or mixtures of carbon monoxide and carbon dioxide are known *per se*, for example a low-pressure synthesis, a medium-pressure-synthesis and a high-pressure synthesis.

i) Low-pressure synthesis

The low-pressure synthesis is generally carried out at pressures between 50 and 100 bar. The temperature is generally 220 to 300°C. As a catalyst, generally a catalyst based on Cu, Zn and $Al_3O_3$ (e.g. $CuO/ZnO/Al_3O_3$) is used. The

low-pressure synthesis is the most preferred synthesis for the preparation of methanol from carbon monoxide or from mixtures of carbon monoxide and carbon dioxide.

ii) Medium-pressure-synthesis

The medium-pressure-synthesis is generally carried out at pressures between 100 and 250 bar. The temperature is generally up to 300°C. As catalysts, generally a catalyst based on $Zn/Cr_2O_3$ or Zn-Cu catalysts are used.

iii) High-pressure synthesis

The high-pressure-synthesis is generally carried out at pressures between 250 and 350 bar. The temperature is generally 320 to 380°C. As a catalysts, generally a catalyst based on zinc-chromium oxide is used. This process is less preferred for the production of methanol from carbon monoxide or from mixtures of carbon monoxide and carbon dioxide.

The current world energy system is still mainly based on the use of fossil fuels and, although the use of renewable energy sources has increased, it will continue in the medium and short term. The massive use of fossil fuels in industry and transport produce large amounts of $CO_2$ emissions. Since it is an object of the present invention to provide environmentally friendly ethylene glycols, selected from mono ethylene glycol, oligo ethylene glycols, poly ethylene glycols, and mixtures thereof, and alkanol ethoxylates, and an environmentally friendly process for making the same, it is preferred that methanol is prepared by reacting the hydrogen from step (a) with carbon dioxide in step (c) according to the process of the present invention.

In step (c) the carbon dioxide and hydrogen are reacted to form methanol.

Process conditions for the hydrogenation of carbon dioxide are known *per se*. Different process approaches are being developed for the synthesis of methanol by hydrogenation of $CO_2$: (1) heterogeneous catalysis, (2), homogeneous catalysis, (3) electrochemical, and (4) photocatalysis (see R. Guil-L6pez, Materials 2019, 12, 3902; doi:10.3390/ma12233902). Preferably, the synthesis of methanol by hydrogenation of carbon dioxide is performed in the presence of a heterogeneous catalyst.

Generally, the methanol production is carried out in a synthesis converter, e.g. a fixed-bed, catalytic reactor.

The average temperature inside the reactor is generally in the range of 150 to 300°C. The average pressure inside the reactor is generally in the range of 50 to 150 bar (abs.).

An overview of suitable heterogeneous catalyst systems is given by Kristian Stange-land, Hailong Li & Zhixin Yu, Energy, Ecology and Environment volume 5, pages 272-285 (2020). Multi-component catalyst systems are required for this process. The interaction between components is essential for high activity and selectivity of CO2-to-methanol catalysts. This has been demonstrated by numerous catalyst systems comprised of various metals (i.e., Cu, Pd, Ni) and metal oxides (i.e.$Al_2O_3$, ZnO, $ZrO_2$, $In_2O_3$). These complex systems can contain a mixture of metallic, alloy, and metal oxide phases. The most promising catalyst systems for large-scale industrial processes are currently Cu-based and In-based catalysts due to their superior catalytic performance. A suitable catalyst is for example copper-zinc-alumina.

By performing step (c), methanol is formed, $CH_3$, by reacting carbon oxides, preferably carbon dioxide, with the hydrogen from step (a). The deuterium content is even lower than corresponding to the distribution obtained by classical petrochemical routes.

In a preferred embodiment the steps (a), (b) and (c) are carried out as described in the International Application with the Application No. PCT/EP2023/060570 filed on 24 April 2023.

Step (d)

In step (d), methanol from step (c) is converted to ethylene and further to ethylene oxide.

Preferably, the ethylene oxide in step (d) is obtained by

(d1) a methanol-to-olefin process, wherein ethylene is obtained, followed by
(d2) epoxidation of ethylene.

Step (d1)

In general, ethylene is produced from methanol in a methanol to olefin-process (MTO-process). The MTO process is an acid catalyzed reaction. Preferred catalysts are zeolites like zeolites containing silica and alumina (e.g. ZSM-5) and silicon alumina phosphate zeolite-catalysts (SAPO) (e.g. SAPO-34).

This reaction is generally carried out at temperatures of from 300-600 °C. The pressure is generally 0.1-0.3 MPa.

The process is preferably carried out in a fluidized catalytic reactor.

The ratio propylene to ethylene can be adjusted by choosing appropriate process conditions, and may vary from

0.77 in the ethylene production mode and 1.33 in the propylene production mode.

Examples for commercial MTO technology licensors are UOP (e.g. UOP Advanced MTO process), Energy Technology Co. Ltd. (DMTO process) and Sinopec (SMTO process).

More detailed descriptions can be found e.g. in "Ethylene" by Adam Chan, Nexant, TECH 2018-1, July 2018, p. 100- 109.

Step (d2)

In step (d2), the ethylene from step (d1) is converted to ethylene oxide.

The direct oxidation process is preferably performed in gas-phase, for example with oxygen or air, in the presence of a catalyst, preferably a silver catalyst, more preferably a silver catalyst supported on alumina.

The step (d2) is generally performed at a temperature of from 230 to 270°C. The pressure is preferably in the range of from 10 to 30 bar.

In a preferred embodiment, step (d2) is performed by gas-phase selective ethylene oxidation (ethylene epoxidation) that is typically performed in fixed-bed tubular reactors with supported Ag/ $Al_2O_3$ catalysts at 230-270 °C and 10-30 bar.

Preferred catalysts for the process in step (d2) are silver-based catalysts like

- supported Re/Cs/Ag/$Al_2O_3$ catalysts that operate preferably in excess $C_2H_4/O_2$; or
- alkaline-metal (Na, Cs)-promoted supported Ag/$Al_2O_3$ catalysts that operate preferably in excess $O_2/C_2H_4$.

Oxides of Mo and S have been found to also promote the supported Re/Cs/Ag/$Al_2O_3$ system for EO formation. Therefore, the supported Re/Cs/Ag/$Al_2O_3$ system may additionally comprise oxides of Mo and/or S as promoters.

In addition, $C_3H_4Cl_2$ may also be added to deposit Cl on the catalyst, which acts as a promoter.

An example for a description can be found e.g. in "Ethylene Oxide" by Mia Monconduit and Karen Jobes, IHS Markit, Chemical Economics Handbook, 22 December 2020, p. 14 - 16.

Step (e)

In step (e1), water with ethylene oxide from step (d) is reacted to ethylene glycols in one or more steps, respectively in step (e2) alkanols $C_nH_{2n+1}$-OH are reacted with ethylene oxide from step (d) to alkanol ethoxylates.

Step (e1)

Ethylene glycol is generally prepared by the non-catalytic reaction of ethylene oxide and water, and is carried out at a reaction temperature of 100-200 °C and at a reaction pressure of 1-30 atmospheres. Under most reaction conditions, the conversion of ethylene oxide, which is the reactant, is 100%, and the reaction product occupies a majority of monoethylene glycol (MEG), diethylene glycol (DEG), triethylene glycol (TEG), and poly ethylene glycols (PEG). The distribution of the product appearing in the process for the production of ethylene glycol undergoing non-catalysis is determined by the constitution ratio of ethylene oxide and water used in the reaction, and generally increases the amount of water, the production of higher ethylene glycols is inhibited and the production of lower ethylene glycol is increased.

Ethylene glycol may also be a by-product of ethylene oxide formation during the manufacturing process.

Commercial production of ethylene glycol generally proceeds in the range of an ethylene oxide: water molar ratio in the range of 1:6 - 1:30, and when increasing the amount of water to maximize production of monoethylene glycol, the cost of purification of monoethylene glycol is increased. In one example in a commercially operated continuous flow reactor, when the reactant ethylene oxide: water molar ratio of 1:22, the resulting ethylene glycol distribution comprises 90% by weight of monoethylene glycol, 9.5% by weight of diethylene glycol, triethylene glycol 0.5%, wherein about 6 tons of water must be removed from the clarifier to produce 1 ton of ethylene glycol (U. S. Patent 3,629,343).

In the process for producing ethylene glycol through catalytic free hydration of ethylene oxide, there has been a problem that the purification cost of ethylene glycol is greatly increased by using excess water to maximize production of monoethylene glycol.

In order to solve the problem of a process for producing ethylene glycol by catalyst-free hydration of ethylene oxide, a process for producing ethylene glycol which is subjected to a reaction apparatus other than ethylene oxide hydration has been developed, the process uses the reactants ethylene oxide and carbon dioxide in addition to water to produce ethylene carbonate, which is hydrated and decarbonized again to produce ethylene glycol. In this case, it is known that the ethylene oxide: water ratio can be reduced to 1: 4 and the cost of purification of ethylene glycol can be reduced.

The carbon dioxide used in the reaction in the process is recovered and used again, and therefore there is no amount consumed as a whole. Catalysts effective for producing ethylene carbonate as intermediates in the production of

ethylene glycol include alkali metal halides (U.S. Patent 3,629,343), Quaternary ammonia halides (British Patent 1,777,877), quaternary amines (German OLS 2,615,595), quaternary phosphates (U.S. Patent 4,160,116), anion exchange resins (U.S. Patent 4,233,221), and the like.

However, this method involves many problems such as discoloration of the product by catalyst, odour, and corrosion of the reactor by carbon dioxide.

The water used in step (e1) is common water and is usually not obtained by hydrogenation of oxygen using low-deuterium hydrogen. Hence, the water used in step (e1) usually exhibits the average deuterium content (molar share of deuterium) of about 150 ppm, based on the total hydrogen content. Therefore, the contribution of hydrogen to the deuterium content of the ethylene glycols of the general formula (I) is 150 ppm for its two hydrogen atoms, while the other hydrogen atoms introduced into the ethylene glycols via ethylene oxide have a lower molar share of deuterium of $\leq$ 100 ppm, preferably in the range of from 10 to $\leq$ 95 ppm, more preferably in the range of from 10 to $\leq$ 90 ppm, most preferably in the range of from 10 to $\leq$ 80 ppm.

Therefore, another subject matter of the present invention are ethylene glycols, obtainable by a reaction as described above.

Step (e2)

There is no specific requirement on the process for obtaining the ethoxylated compounds, and the preparation of the alkanol ethoxylates of the present invention is generally known by a person skilled in the art.

The ethoxylation can generally be carried out in three ways: (i) anionic (base-initiated) polymerization, (ii) acid initiated polymerization, and (iii) by coordination polymerization.

The anionic polymerization of epoxides represents the "classical" technique for the synthesis of the respective polymers/compounds comprising ethylene oxide units. The anionic polymerization is usually carried out by catalytic addition of ethylene oxide onto at least one alkanol $H_{2n+1}C_n$-OH.

As catalysts, metal compounds, preferably alkali metal (especially sodium, potassium, or cesium) compounds with high nucleophilicity can be employed. Examples are alkali metal hydroxides, alkali metal salts, alkali metal hydrides, or alkali metal amides. Potassium hydroxide having the greatest significance in practice (see for example US 6156720 A).

A further suitable class of catalyst are multimetal cyanide compounds, preferably double metal cyanide compounds, especially zinc hexacyanometalates. These catalysts are frequently also referred to as DMC catalysts. The polyether alcohols prepared using multimetal cyanide compounds feature a very low content of unsaturated constituents. A further advantage in the use of multimetal cyanide compounds as catalysts consists in the distinctly increased space-time yield in the addition of the alkylene oxides. The alkoxylation in the presence of DMC catalysts is for example described in DD 203 735, DD 203 734, WO 97/29146, WO 98/03571, WO 00/14143, WO 99/44739 and US 2008/0161509 A1.

Solvents employed for the anionic polymerization of epoxides are generally polar and aprotic; therefore, tetrahydrofuran (THF), dioxane, dimethyl sulfoxide (DMSO), and hexamethylphosphoramide (HMPA) are often used. Furthermore, polymerization in the bulk monomer is possible and is the preferred process.

Alkoxides with sodium, potassium, or cesium counterions in THF or other polar, aprotic solvents represent popular initiator systems.

The addition of complexing agents, such as crown ethers suitable for the respective cation can strongly accelerate the anionic polymerization of epoxides.

The temperatures during the alkoxylation are usually between 80 and 200°C, preferably 90 to 180°C.

The ethoxylated compounds of the present invention can be prepared either in a batchwise, semibatchwise or in a continuous process.

In a semibatch ethoxylation, for example, the catalyst and the at least one alkanol are initially charged while ethylene oxide is added during the reaction course. This particular synthesis strategy is due to the high reactivity of alkoxides and also to the high heat involved in ethoxylation reaction.

The polymerization rate of ethylene oxide (EO) is considerably faster than that of propylene oxide (PO), which plays an important role in the frequently used anionic copolymerization of EO and PO. Generally, the reactivity of alkylene oxides decreases with increasing length and bulkiness of the alkyl substituent at the epoxide moiety.

In a preferred embodiment the ethoxylation is carried out continuously and more preferably continuously in a tube reactor. Such reaction usually leads to a narrow molecular weight distribution and less formation of byproducts.

Therefore, another subject matter of the present invention are alkanol ethoxylates, obtainable by a reaction as described above.

In the case of alkanol ethoxylates, they can be converted into alkyl ether sulfate salts by sulfating them in a manner known per se using sulfuric acid or sulfuric acid derivatives to give acid alkyl ether sulfate salts (see for example US 2008/0207939 A1). Sulfation reactions of alcohols have already been described, for example in US 3,462,525, US 3,420,875 and US 3,524,864. Details on carrying out this reaction are also given in "Ullmann's Encyclopedia of Industrial

Chemistry", 5th edition, Vol. A25 (1994), pages 779-783 and in the literature references given there. Thus, non-ionic detergents can be converted into ionic detergents.

If sulfuric acid itself is used for the esterification, expediently use is generally made of from 75 to 100% strength by weight, preferably from 85 to 98% strength by weight, acid (termed "concentrated sulfuric acid" or "monohydrate". The esterification can be formed in a solvent or diluent if it is wanted for control of the reaction, for example heat development.

Generally, the alcoholic reactant is introduced first and the sulfation reagent is added gradually with continuous mixing. If complete esterification of the alcohol alkoxide component is desired, the sulfation reagent and the alcohol alkoxide component are generally used in a molar ratio of from 1:1 to 1:1.5, preferably from 1:1 to 1:1.2. Smaller amounts of sulfation reagent can be advantageous if mixtures of alcohol alkoxylates are used. The esterification is usually carried out at temperatures of from 25 to 85° C, preferably in the range from 45 to 75° C. If appropriate it can be expedient to carry out the esterification in a low-boiling, water-immiscible solvent and diluent at its boiling point, the water being formed in the esterification being distilled off azeotropically.

Instead of sulfuric acid of the concentration stated above, for the sulfation of the inventive alcohol alkoxide component, use can also be made, for example, of sulfur trioxide, sulfur trioxide complexes, solutions of sulfur trioxide in sulfuric acid ("oleum"), chlorosulfonic acid, sulfuryl chloride or else sulfamic acid. The reaction conditions must then be modified appropriately as known by a person skilled in the art.

If sulfur trioxide is used as sulfation reagent, the reaction can also be carried out advantageously in a falling-film reactor in countercurrent or cocurrent flow, if appropriate also continuously. The batches, after the esterification, are neutralized by adding alkali and, if appropriate after removing excess alkali metal sulfate and any solvent present, are worked up.

If chlorosulfonic acid is used as sulfating reagent, the corresponding alcohol alkoxide component is charged into a stirred apparatus under inert conditions. Under vigorous stirring, a corresponding amount of chlorosulfonic acid is added dropwise. The molar ratio between alcohol component and chlorosulfonic acid is generally from 0.5:1 to 1:0.5, preferably the ratio is from 0.75:1 to 1:0.75. Very particularly preferably, the molar ratio of alcohol alkoxide component to chlorosulfonic acid is 1:1. After the HCl gas is removed, the reaction batch is adjusted to a slightly alkaline pH using sodium hydroxide solution.

In the case of alkanol ethoxylates, they can be converted into polyetheramines by subsequent reductive amination with ammonia, monoamines or polyamines. Such products are described more particularly in EP-A 310 875, EP-A 356 725, EP-A 700 985 and US-A 4,877,416. For example, the polyetheramines used may be poly-$C_2$- to $C_6$-alkylene oxide amines or functional derivatives thereof. Typical examples thereof are tridecanol ethoxylates or isotridecanol ethoxylates, heptadecanol ethoxylates or isoheptadecanol ethoxylates, isononylphenol ethoxylates and also polyisobutenol ethoxylates.

In a preferred embodiment the ammonia used for such a reductive amination exhibits a molar share of deuterium which is not more than 100 ppm, preferably from 10 to 98, more preferably from 10 to 95m and even more preferably from 10 to 90 ppm.

A process for production of such ammonia is disclosed in the International Application with the Application No. PCT/EP2023/061271 and the application date 28 April 2023.

Alkanols $H_{2n+1}C_n$-OH for use in step (e2) comprise 1 to 20 carbon atoms, may be linear or branched and may originated from fossil or renewable sources.

Examples for such alkanols are methanol, ethanol, n-propanol, iso-propanol, n-butanol, iso-butanol, hexanol, n-octanol, 2-ethyl hexanol, iso-nonanol, n-decanol, 2-propyl heptanol, 2-butyloctanol, $C_{12}$ alcohol, $C_{13}$ alcohol, $C_{14}$ alcohol, $C_{15}$ alcohol, $C_{16}$ alcohol, $C_{17}$ alcohol, $C_{18}$ alcohol (e.g. stearyl alcohol), oleyl alcohol, isononadecanol, eicosanol, and mixtures of said alcohols like $C_{13}$-$C_{15}$ alcohol, $C_{12}$-$C_{18}$ alcohol, $C_{16}$-$C_{18}$ alcohol, or $C_{12}$-$C_{14}$ alcohol.

The alkanol used in step (e2) is usually not obtained by hydrogenation using low-deuterium hydrogen. Hence, the alkanol used in step (e2) usually exhibits the average deuterium content (molar share of deuterium) of about 150 ppm, based on the total hydrogen content. Therefore, the contribution of hydrogen to the deuterium content of the alkanol ethoxylates of the general formula (II) is 150 ppm for its (2n + 2) hydrogen atoms, while the other hydrogen atoms introduced into the ethylene glycols via ethylene oxide have a lower molar share of deuterium of $\leq$ 100 ppm, preferably in the range of from 10 to $\leq$ 95 ppm, more preferably in the range of from 10 to $\leq$ 90 ppm, most preferably in the range of from 10 to $\leq$ 80 ppm.

In those cases in which the alkanol with the formula $H_{2n+1}C_n$-OH used in step (e2) is obtained via hydrogenation of a carboxylic acid or a carboxylic acid ester or an aldehyde which in turn had been obtained by hydroformylation using low-deuterium hydrogen the molar share of deuterium may be further reduced:

- In case of hydroformylation using low-deuterium hydrogen the molar share of the deuterium in the thus obtained alkanol is $\leq$ (2n) $\times$ 150 ppm + 2 $\times$ 100 ppm

- In case of hydrogenation of an aldehyde using low-deuterium hydrogen the molar share of the deuterium in the thus

obtained alkanol is ≤ (2n) × 150 ppm + 2 × 100 ppm

- In case of hydroformylation using low-deuterium hydrogen followed by hydrogenation of an aldehyde using low-deuterium hydrogen the molar share of the deuterium in the thus obtained alkanol is ≤ (2n-2) × 150 ppm + 4 × 100 ppm
- In case of hydrogenation of a carboxylic acid (ester) using low-deuterium hydrogen the molar share of the deuterium in the thus obtained alkanol is ≤ (2n-2) × 150 ppm + 4 × 100 ppm

In case hydrogen with a lower share of deuterium is used, e.g. in the range of from 10 to ≤ 95 ppm, more preferably in the range of from 10 to ≤ 90 ppm, most preferably in the range of from 10 to ≤ 80 ppm, these values are to be used in the above-mentioned calculations instead of 100 ppm.
Suitable alkanols which may be obtained from hydroformylation and/or hydrogenation are e.g. n-butanol, iso-butanol, 2-ethyl hexanol, iso-nonanol, 2-propyl heptanol, 2-butyloctanol, $C_{13}$ alcohol, $C_{15}$ alcohol, and $C_{17}$ alcohol.
In an exceptional case in step (e2) methanol or ethanol with a low molar share of deuterium can be used as alkanols if methanol from step (c) is used or ethanol obtained by hydrogenation of ethylene oxide from step (d) with hydrogen with a low molar share of deuterium obtained from step (a).
In these exceptional cases the following alkanol ethoxylates are obtainable:

(IIa) $H_3C\text{-}O\text{-}[\text{-}CH_2\text{-}CH_2\text{-}O\text{-}]_m\text{-}H$ with a molar share of deuterium of not more than 100 ppm, preferably 10 to 98 ppm, more preferably 10 to 95, and even more preferably 10 to 90 ppm.

(lib) $H_5C_2\text{-}O\text{-}[\text{-}CH_2\text{-}CH_2\text{-}O\text{-}]_m\text{-}H$ with a molar share of deuterium of not more than 100 ppm, preferably 10 to 98 ppm, more preferably 10 to 95, and even more preferably 10 to 90 ppm.

## Coolants

Another subject matter of the present invention are coolants, comprising at least one ethylene glycol according to the present invention, preferably mono ethylene glycol as freezing point depressant.
Preferred coolants comprise further constituents as follows

(A) at least one ethylene glycol
(B) water
(C) at least one azole derivative
(D) optionally at least one ester of orthosilicic acid or alkoxy alkylsilane
(E) optionally at least one inorganic salt selected from the group consisting of molybdates, borates, phosphates, silicates, nitrites and nitrates,
(F) optionally at least one mono- or dicarboxylic acid
(G) optionally at least one silicophosphonate
(H) optionally at least one tertiary amine as corrosion inhibitor
(I) optionally at least one further coolant additive,

wherein
at least one inorganic salt and/or at least one carboxylic acid is present.
Details to the constituents are as follows:

### Glycol (A)

As freezing point depressant (A) at least on ethylene glycol component or derivative thereof according to the present invention having a low molar share of deuterium is used, in particular, monoethylene glycol, diethylene glycol, triethylene glycol, tetraethylene glycol and mixtures thereof, monoethylene glycol monomethyl ether, diethylene glycol monomethyl ether, triethylene glycol monomethyl ether, tetraethylene glycol monomethyl ether, monoethylene glycol monoethyl ether, diethylene glycol monoethyl ether, triethylene glycol monoethyl ether, tetraethylene glycol monoethyl ether, monoethylene glycol mono-n-butyl ether, diethylene glycol mono-n-butyl ether, triethylene glycol mono-n-butyl ether and tetraethylene glycol mono-n-butyl ether, in each case either alone or as mixtures thereof.
In a preferred embodiment monoethylene glycol with a molar share of deuterium of not more than 117 ppm, preferably from 10 to 115, more preferably from 10 to 113, and especially from 10 to 110 ppm is used in the coolants according to the present invention.

Water (B)

Water used for the coolants should be ion-free, designating water with a neutral pH-value and comprising essentially no further ions than those hydroxide ions and hydronium ions out of the autoprotolysis of water at the respective temperature.

In a preferred embodiment, especially if a coolant with a low electrical conductivity is aimed for, the electrical conductivity (throughout this text determined according to ASTM D 1125) at 20 °C of the ion-free water used should preferably not exceed 5 μS/cm, more preferably not more than 3, even more preferably not more than 2, and especially not more than 1 μS/cm.

The ion-free water used can be pure distilled or twice-distilled water or water which has been deionized, for example by ion exchange.

For conventional coolants for which the electrical conductivity is less important, also hard water can be used. In order to enable the use of hard water, at least one hard water stabilizer can be added to the coolant composition.

The water used may contain alkaline earth metal ions, such as magnesium, calcium, strontium or barium ions, although the latter are usually only contained in trace amounts. Preferably, only magnesium and/or calcium ions are contained as hardening agents.

The water used is preferably soft water with a water hardness of no more than 8.4 °dH, preferably no more than 10 ° and preferably no more than 12 °dH.

When using water with a water hardness of up to 14 °dH, preferably up to 17, especially preferably up to 20 and even up to 25 °dH, a hard water stabilizer is usually required.

The water used in the coolant is the usual source of any carbonate and/or sulphate contained in the coolant via the hardening agents.

Azole Derivatives (C)

Azole derivatives in the context of the present invention mean five-membered heterocyclic compounds having 2 or 3 heteroatoms from the group consisting of nitrogen and sulfur and comprise no or at most one sulfur atom and can bear an aromatic or saturated six-membered fused-on ring.

These five-membered heterocyclic compounds (azole derivatives) usually contain two N atoms and no S atom, 3 N atoms and no S atom or one N atom and one S atom as heteroatoms.

Preferred groups of the specified azole derivatives are annellated imidazoles and annellated 1,2,3-triazoles of the general formula

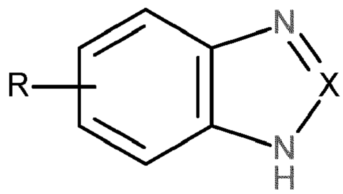

(I)

or

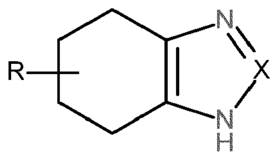

(II)

where

the variable R is hydrogen or a $C_1$-$C_{10}$-alkyl radical, in particular methyl or ethyl, and
the variable X is a nitrogen atom or the C-H group.

Typical and preferred examples of azole derivatives of the general formula (I) are benzimidazole (X = C-H, R = H), benzotriazoles (X = N, R = H) and tolutriazole (tolyltriazole) (X = N, R = $CH_3$). A typical example of an azole derivative of the

general formula (II) is hydrogenated 1,2,3-tolutriazole (tolyltriazole) (X = N, R = $CH_3$).

A further preferred group of the specified azole derivatives is benzothiazoles of the general formula (III)

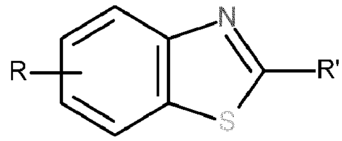

where

the variable R is as defined above and

the variable R' is hydrogen, a $C_1$-$C_{10}$-alkyl radical, in particular methyl or ethyl, or in particular a mercapto group (-SH).

A typical example of an azole derivative of the general formula (III) is 2-mercaptobenzothiazole.

It is also possible, however less preferable, to use (2-benzothiazylthio)acetic acid (R' = -S-$CH_2$-COOH) or (2-benzothiazylthio) propionic acid (R' = -S-$CH_2$-$CH_2$-COOH). This embodiment is less preferable since the use of such free-acid compounds would increase the electrical conductivity of the coolant.

Further suitable azole derivatives are non-annellated azole derivatives of the general formula (IV)

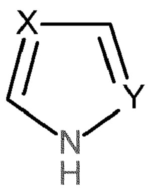

(IV)

where

the variables X and Y together are two nitrogen atoms or

one nitrogen atom and a C-H group,

for example 1 H-1 ,2,4-triazole (X = Y = N) or preferably imidazole (X = N, Y = C-H).

For the purposes of the present invention, benzimidazole, benzotriazole, tolutriazole, hydrogenated tolutriazole or mixtures thereof, in particular benzotriazole or tolutriazole, are very particularly preferred as azole derivatives.

The azole derivatives mentioned are commercially available or can be prepared by conventional methods. Hydrogenated benzotriazoles such as hydrogenated tolutriazole are likewise obtainable as described in DE-A 1 948 794 and are also commercially available.

Esters of Orthosilicic Acid or Alkoxy Alkylsilanes (D)

Esters of orthosilicic acid are compounds of the formula

$$Si(OR^1)_4$$

wherein

$R^1$ is an organic substituent comprising 1 to 6 carbon atoms, for example a linear or branched, preferably a linear alkyl substituent comprising 1 to 6 carbon atoms or an aromatic substituent comprising 6 carbon atoms, more preferably an alkyl substituent comprising 1 to 4 carbon atoms and even more preferably an alkyl substituent comprising 1 or 2 carbon atoms.

Alkoxy alkylsilanes are less preferred and both the alkoxy substituent as well as the alkyl group comprise a linear or branched, preferably a linear alkyl substituent comprising 1 to 6 carbon atoms, more preferably an alkyl substituent comprising 1 to 4 carbon atoms and even more preferably an alkyl substituent comprising 1 or 2 carbon atoms.

Typical examples of compounds (D) are tetraalkoxysilanes, preferably tetramethoxysilane and tetraethoxysilane, and alkoxyalkylsilanes, preferably triethoxymethylsilane, diethoxydimethylsilane, ethoxytrimethylsilane, trimethoxymethylsilane, dimethoxydimethylsilane and methoxytrimethylsilane. Preference is given to tetraalkoxysilanes, particularly preferably tetramethoxysilane and tetraethoxysilane, with very particular preference being given to tetraethoxysilane.

Compounds (D) are mainly used as inhibitors of aluminium corrosion.

Inorganic Salt (E)

The inorganic salts are often used as corrosion inhibitors against the corrosion of materials containing iron and/or aluminium as well as solder.
The least one inorganic salt is selected from the group consisting of molybdates, borates, phosphates, silicates, nitrites and nitrates, preferably selected from the group consisting of borates, phosphates, silicates, nitrites and nitrates, especially preferentially selected from the group consisting of phosphates, silicates, and nitrates.
Inorganic inhibitors are phosphates, silicates, borates, nitrites, nitrates or molybdates, or mixtures thereof in the form of their free acids or their salts, especially their alkali metal salts, especially their sodium or potassium salts. The form in which they are present (protonated or as salt) in the compositions, superconcentrates, concentrates or coolants depends on the respective pKs value of the compound and the composition as well as the pH of the respective environment, which is determined by the amount of base.
The molybdates are used as free acid ($H_2MoO_4$), as hydrogen molybdate or preferably as molybdate, especially as alkali metal salts, especially as sodium or potash salts and especially as sodium salts. The acidic protons in the molybdate may be partially or completely replaced by alkali metal salts. The use of sodium molybdate, usually in the form of dihydrate, is preferred.
Borates are preferably used as sodium tetraborate (borax) or as potassium tetraborate, especially as sodium tetraborate.
The phosphates are used as free acid ($H_3PO_4$), hydrogen phosphate, dihydrogen phosphate or phosphate, especially as alkali metal salts, especially as sodium or potassium salts. The acidic protons in the phosphates can be partially or completely replaced by alkali metal salts.
It is also conceivable to use the corresponding diphosphates, triphosphates or oligophosphates, also in mixtures with monophosphates, but they are preferably used as monomeric phosphates.
It is preferred to be used as a free acid ($H_3PO_4$), disodium hydrogen phosphate or trisodium phosphate.
The inorganic silicates mainly act as inhibitors of corrosion of aluminium and aluminium-containing materials as well as solder and are mostly used as alkali metal salts or, less frequently, as magnesium, calcium or aluminium salts, preferably as sodium or potassium salts.
The silicates are preferably selected from the group consisting of orthosilicates ($SiO_4^{4-}$), metasilicates ($SiO_3^{2-}$), and pyrosilicates ($Si_2O_7^{6-}$), especially methasilicates ($SiO_3^{2-}$), especially sodium metasilicate ($Na_2SiO_3$) or potassium metasilicate ($K_2SiO_3$), especially sodium metasilicate ($Na_2SiO_3$).
The nitrites are all inorganic salts of the nitrite anion ($NO_2^-$) and nitric acid ($HNO_2$). Typically, nitrite is used in the form of sodium nitrite or potassium nitrite in coolants or is converted into them by the inorganic bases, preferably sodium hydroxide or potassium hydroxide.
In a preferred embodiment, the coolants do not contain nitrite for health reasons.
The nitrates are used as alkali or alkaline earth metal nitrates, preferably as sodium nitrate, potassium nitrate or magnesium nitrate, preferably as sodium nitrate or potassium nitrate, especially as sodium nitrate.
Preferred inorganic salts are selected from the group consisting of molybdates, borates, phosphates and nitrates, especially selected from the group consisting of molybdates, phosphates and silicates.

Carboxylic Acid (F)

Suitable monocarboxylic acids (F) may be linear or branched-chain, aliphatic, cycloaliphatic or aromatic monocarboxylic acids with up to 20 carbon atoms, preferably with from 2 to 18, more preferably with from 5 to 16, even more preferably with from 5 to 14, most preferably with from 6 to 12, and especially with from 8 to 10 carbon atoms.
Branched-chain aliphatic monocarboxylic acids are preferred over the corresponding linear monocarboxylic acids.
Useful linear or branched-chain, aliphatic or cycloaliphatic monocarboxylic acids (F) are, for example, propionic acid, pentanoic acid, 2,2-dimethylpropanoic acid, hexanoic acid, 2,2-dimethylbutaneoic acid, cyclohexyl acetic acid, octanoic acid, 2-ethylhexanoic acid, nonanoic acid, isononanoic acid, decanoic acid, undecanoic acid or dodecanoic acid.
A suitable aromatic monocarboxylic acid (F) is in particular benzoic acid; additionally useful are also, for example, $C_1$- to Cs-alkylbenzoic acids such as o-, m-, p-methylbenzoic acid or p-tert-butylbenzoic acid, and hydroxyl-containing aromatic monocarboxylic acids such as o-, m- or p-hydroxybenzoic acid, o-, m- or p-(hydroxymethyl)benzoic acid or halobenzoic acids such as o-, m- or p-fluorobenzoic acid.
Especially preferred are 2-ethylhexanoic acid and isononanoic acid, with isononanoic acid being most preferred.
As used herein, isononanoic acid refers to one or more branched-chain aliphatic carboxylic acids with 9 carbon atoms. Embodiments of isononanoic acid used in the engine coolant composition may include 7-methyloctanoic acid (e.g., CAS Nos. 693-19-6 and 26896-18-4), 6,6-dimethylheptanoic acid (e.g., CAS No. 15898-92-7), 3,5,5-trimethylhexanoic acid (e.g., CAS No. 3302-10-1), 3,4,5-trimethylhexanoic acid, 2,5,5-trimethylhexanoic acid, 2,2,4,4-tetramethylpentanoic

acid (e.g., CAS No. 3302-12-3) and combinations thereof. In a preferred embodiment, isononanoic acid has as its main component greater than 90% of one of 7-methyloctanoic acid, 6,6-dimethylheptanoic acid, 3,5,5-trimethylhexanoic acid, 3,4,5-trimethylhexanoic acid, 2,5,5-trimethylhexanoic acid, and 2,2,4,4-tetramethylpentanoic acid. The balance of the isononanoic acid may include other nine carbon carboxylic acid isomers and minor amounts of one or more contaminants. In a preferred embodiment, the isononanoic acid has as its main component greater than 90% of 3,5,5-trimethylhexanoic acid and even more preferably, the main component is greater than 95% 3,5,5-trimethylhexanoic acid.

It is possible to use carboxylic acids with a higher functionality, e.g. di- or tricarboxylic acids, in addition to or instead of the monocarboxylic acids.

If used, di- or tricarboxylic acids can be aliphatic, cycloaliphatic or aromatic, preferably aliphatic or aromatic and more preferably aliphatic with up to 20 carbon atoms, preferably with up to 18, more preferably with up to 16, even more preferably with up to 14, and especially up to 12 carbon atoms.

If used, examples of dicarboxylic acids are oxalic acid, malonic acid, succinic acid, glutaric acid, adipic acid, pimelic acid, suberic acid, azelaic acid, sebacic acid, undecanedioic acid, dodecanedioic acid, alkyl or alkenyl succinic acids, 2-metylbutane dioic acid, 2-ethylpentanedioic acid, 2-n-dodecylbutanedioic acid, 2-ndodecenylbutanedioic acid, 2-phenylbutanedioic acid, 2-(p-methylphenyl) butanedioic acid, 2,2-dimethylbutanedioic acid, 2,3-dimethylbutanedioic acid; 2,3,4 trimethylpentanedioic acid, 2,2,3-trimethylpentanedioic acid; 2-ethyl-3-methylbutanedioic maleic acid, fumaric acid, pent-2-enedioic acid, hex-2-enedioic acid; hex-3-endioic acid; 5-methylhex-2-enedioic acid; 2,3-dimethylpent-2-enedioic acid; 2-methylbut-2-enedioic acid, 2-dodecylbut-2-enedioic acid, phthalic acid, isophthalic acid, terephthalic acid and substituted phthalic acids such as 3-methylbenzene-1,2-dicarboxylic acid; 4-phenylbenzene-1,3-dicarboxylic acid; 2-(1-propenyl) benzene-1,4-dicarboxylic acid, and 3,4-dimethylbenzene-1,2-dicarboxylic acid.

If used, examples of tricarboxylic acids are benzene tricarboxylic acids (all isomers) and triazinetriiminocarboxylic acids such as 6,6',6"-(1,3,5-triazine-2,4,6-triyltriimino)trihexanoic acid.

Silicophosphonate (G)

As an optional constituent it is possible to use at least one silicophosphonate (G) in the coolant according to the invention.

Silicophosphonates are those of the general structure (V)

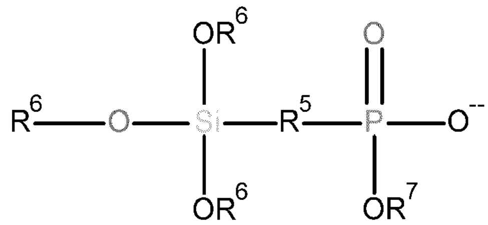

where

R⁵ is a bivalent organic residue, preferably a 1,ω-alkylene group with 1 to 6, preferably 1 to 4 carbon atoms, more preferably methylene, 1,2-ethylene, 1,2-propylene, 1,3-propylene or 1,4-butylene, most preferably 1,2-ethylene or 1,3-propylene, and especially 1,2-ethylene,

$R^6$ and $R^7$ are independently of another $C_1$- to $C_4$-alkyl, preferably methyl, ethyl, n-propyl, isopropyl, n-butyl, iso-butyl, sec-butyl or tert-butyl, preferably methyl or ethyl.

Such silicophosphonates may exist as free phosphonate acid or in the form of their sodium or potassium salts, preferably sodium or potassium salt, more preferably as sodium salt.

Tertiary Amines (H)

The at least one tertiary amine (H) as corrosion inhibitor bears at least one 2-hydroxyethyl- or 2-hydroxypropyl-group. Preferred tertiary amines (H) may bear one, two or three 2-hydroxyethyl- or 2-hydroxypropyl-groups, preferably two or three 2-hydroxyethyl- or 2-hydroxypropyl-groups and more preferably 2-hydroxyethyl-groups.

The substituents of the tertiary amine (H) not being a 2-hydroxyethyl- or 2-hydroxypropyl-group may be aliphatic, cycloaliphatic or aromatic groups with up to 20 carbon atoms, preferably with up to 18, more preferably with up to 16, even

more preferably with up to 14, and especially up to 12 carbon atoms.

These substituents are preferably aliphatic or aromatic and more preferably aliphatic.

Aliphatic substituents may be linear or branched, preferred are linear alkyl substituents comprising 1 to 18 carbon atoms, preferably 2 to 16, more preferably 4 to 14, and especially 6 to 12 carbon atoms.

Especially preferred are aliphatic substituents with 4 to 10, preferably 6 to 8, more preferably 8 carbon atoms.

Preferred individuals bearing one 2-hydroxyethyl- or 2-hydroxypropyl-group are dimethyl ethanolamine, dimethyl propanolamine, diethyl ethanolamine, diethyl propanolamine, di-n-butyl ethanolamine, di-n-butyl propanolamine, N-hydroxyethyl pyrrolidine, N-hydroxyethyl piperidine, and N-hydroxyethyl morpholine.

Preferred individuals bearing two 2-hydroxyethyl- or 2-hydroxypropyl-groups are the bis(2-hydroxyethyl) amines or bis(2-hydroxypropyl) amines bearing as substituent n-butyl amine, n-hexylamine, 2-methylpentylamine, n-heptylamine, 2-heptylamine, isoheptylamine, 1-methylhexylamine, n-octylamine, 2-ethylhexylamine, 2-aminooctane, 6-methyl-2-heptylamine, n-nonylamine, isononylamine, n-decylamine and 2-propylheptylamine or mixtures thereof.

Particular preference is given to bis(2-hydroxyethyl)-substituted n-butyl amine, n-hexylamine, n-octylamine, 2-ethylhexylamine and n-decylamine, with n-octylamine and 2-ethylhexylamine, in particular bis(2-hydroxyethyl) n-octylamine, being particularly preferred.

Examples for tertiary amines (H) bearing three 2-hydroxyethyl- or 2-hydroxypropyl-groups are triethanolamine and tripropanolamine, preferably triethanolamine.

Although diisopropanol amine is not a tertiary amine it is also a preferred compound (H) useful as corrosion inhibitor.

Preferred amines (H) are dimethyl ethanolamine, dimethyl propanolamine, diethyl ethanolamine, di-n-butyl ethanolamine, N-hydroxyethyl morpholine, bis(2-hydroxyethyl) n-hexylamine, bis(2-hydroxyethyl) n-octylamine, bis(2-hydroxyethyl) 2-ethylhexylamine, bis(2-hydroxyethyl) n-decylamine, triethanolamine, and diisopropanol amine.

In case ethanolamines are used such ethanolamines may also preferably exhibit a low share of deuterium wherein the molar share of deuterium in each $-CH_2-CH_2-OH$ moiety is not more than 100 ppm, preferably from 10 to 98, more preferably from 10 to 95m and even more preferably from 10 to 90 ppm.

A process for production of such ethanolamines is disclosed in the International Application with the Application No. PCT/EP2023/061271 and the application date 28 April 2023.

Further Coolant Additives (I)

It is further possible to add further typical coolant additives to the coolants.

As further customary assistants, the coolant may also comprise, in customary small amounts, defoamers (generally in amounts of from 0.003 to 0.008% by weight) and, for reasons of hygiene and safety in the event that it is swallowed, bitter substances (for example of the denatonium benzoate type) and dyes.

Wherever possible the use of non-ionic additives is preferred over ionic alternatives as long as a similar effect can be achieved using the non-ionic additives.

Composition

Typically, the coolants according to the invention are composed as follows:

(A) at least one glycol: 10 to 90 wt%, preferably 20 to 80 wt%, more preferably 30 to 70 wt%
(B) water: 10 to 90 wt%, preferably 20 to 80 wt%, more preferably 30 to 70 wt%
(C) at least one azole derivative: 0.01 to 1 wt%, preferably 0.02 to 0.9 wt%, more preferably 0.03 to 0.8 wt%, even more preferably 0.04 to 0.5, especially 0.05 to 0.3 wt%
(D) optionally at least one ester of orthosilicic acid or alkoxy alkylsilane: 0.01 to 1 wt%, preferably 0.02 to 0.9 wt%, more preferably 0.03 to 0.8 wt%, even more preferably 0.04 to 0.5, especially 0.05 to 0.3 wt%
(E) optionally at least one inorganic salt: 0 to 5 wt%, preferably 0.25 to 4.5 wt%, more preferably 0.5 to 4.0 wt%
(F) optionally at least one carboxylic acid: 0 to 5 wt%, preferably 0.25 to 4.5 wt%, more preferably 0.5 to 4.0 wt%
(G) optionally at least one silicophosphonate: 0 to 1 wt%, preferably 0.01 to 0.8 wt%, more preferably 0.02 to 0.6 wt%
(H) optionally at least one tertiary amine as corrosion inhibitor: 0 to 1 wt%, preferably 0.01 to 0.9 wt%, more preferably 0.02 to 0.8 wt%
(I) optionally at least on further coolant additive: 0 to 0.5 wt% for each further coolant additive, preferably 0.01 to 0.4 wt%, more preferably 0.02 to 0.3 wt%.

with the proviso that the sum of all components always add up to 100 wt%, wherein at least one inorganic salt and/or at least one carboxylic acid is present.

The coolants according to the present invention may be used in cooling systems of stationary engines or vehicles

with combustion engines, electric engines, fuel cells or hybrid engines with a combination of combustion engines with electric engines or a combination of combustion engines with fuel cells, or in generators, such as wind turbines.

For coolants in which the electrical conductivity is less important, the electrical conductivity of the coolants is usually in the range of from 1 to 6000 μS/cm (determined according to ASTM D 1125 at 20 °C), preferably up to 5000, more preferably up to 4000, and even more preferably up to 3000 μS/cm. This is the preferably case for coolants which are mainly to be used in combustion engines or in generators.

Coolants for use in electric engines, fuel cells or hybrid engines should preferably an electrical conductivity at 20 °C of less than 100, preferably less than 50 μS/cm (determined according to ASTM D 1125) to make the suitable for cooling systems of vehicles with electric engines.

In order to achieve that purpose the amount of ionic species, species which may contain ionic byproducts or combination of species which may form ions, such as acids and bases, should be kept at a minimum in order not to raise the electrical conductivity over the critical value.

Therefore, the amount of components (C) to (I) in the coolant are chosen in a way that the critical value for the electrical conductivity is not exceeded.

Brake Fluids

Functional fluid compositions based on borate esters are well known in the art. To be useful for example as DOT 4 or DOT 5.1 brake fluids, these borate ester based compositions must meet stringent physical properties and performance requirements particularly with respect to minimum dry equilibrium reflux boiling point ("ERBP"), minimum wet equilibrium reflux boiling point ("WERBP") and maximum low temperature kinematic viscosity (e.g. determined at -40°C) while maintaining adequate resistance to corrosion, stability and meeting other physical property requirements such as pH, reserve alkalinity and rubber swell.

Examples of borate containing brake fluids are disclosed in US 6558569 which content is incorporated by reference in this application.

Preferred borate-containing brake fluids comprise

(A) from 15 to 90% by weight, based on the weight of the total composition, of one or more alkoxy glycol borate esters having the general formula (III)

$$[R^1\text{-O-}(CH_2CH_2\text{-O})_x]_3B \qquad \text{(III)}$$

wherein

$R^1$ is a $C_1$- to $C_4$-alkyl radical or a mixture of such radicals and
x has a value of from 2 to 6,

(B) from 5 to 80% by weight, based on the weight of the total composition, of one or more alkoxy glycol components having the general formula (IV)

$$R^2\text{-O-}(CH_2CH_2\text{-O})_y\text{-H} \qquad \text{(IV)}$$

wherein

$R^2$ is a $C_1$- to $C_8$-alkyl radical or a mixture of such radicals and
y has a value of from 2 to 6,
$R^2$ and/or y being different or identical to $R^1$ and/or x, respectively, preferably identical,

(C) from 0.1 to 10% by weight, based on the total weight of the composition, of an additive package comprising one or more additives with corrosion inhibition action, wherein preferably at least one of the additives contained in the additive package of component (C) is selected from alkylamine ethoxylates,

wherein at least one of the alkanol ethoxylates
$R^1$-O-($CH_2CH_2$-O-)-H in component (A) or component (B) is an alkanol ethoxylate having a low molar share of deuterium.

In one embodiment of the present invention the compounds

$R^1$-O-($CH_2CH_2$-O)$_x$-H underlying the compound according to formula (III) as well as
$R^2$-O-($CH_2CH_2$-O)$_y$-H according to formula (IV)
are alkanol ethoxylates according to the present invention, having a low molar share of deuterium.

Component (A) of the functional fluid composition of general formula (III) comprises species of ethoxylation degree of from x = 2 to x = 6, preferably of from x = 2 to x = 4, more preferably of x = 3. Component (A) may be a single species or a mixture of different species with regard to the ethoxylation degree and/or to radical $R^1$. Radical $R^1$ is preferably a $C_1$- to $C_4$-alkyl radical and may be methyl, ethyl, n-propyl, isopropyl, n-butyl, isobutyl, sec-butyl, tert-butyl, n-pentyl, n-hexyl, n-heptyl, n-octyl and 2-ethylhexyl, ethyl and especially methyl being preferred.

The said borate esters and their methods of preparation are well known in the art. Borate esters especially useful in the functional fluid composition of the present invention may be prepared by reacting boric acid with suitable alkoxy glycol components which are different or identical to those of component (B). Typically, such alkoxy glycol components are mixtures of different species with regard to the ethoxylation degree and/or to radical $R^1$.

Examples of useful borate esters include those containing methyl triethylene glycol borate ester which can also be named tris-[2-[2-(2-methoxyethoxy)-ethoxy]-ethyl) orthoborate, ethyl triethylene glycol borate ester, n-butyl triethylene glycol borate ester and mixtures thereof. Further useful borate esters include those containing methyl tetraethylene glycol borate ester, methyl diethylene glycol borate ester, ethyl tetraethylene glycol borate ester, ethyl diethylene glycol borate ester, n-butyl tetraethylene glycol borate ester, n-butyl diethylene glycol borate ester and mixtures thereof.

In a preferred embodiment, component (A) comprises at least on alkoxy glycol borate ester of general formula (III) wherein the ethoxylation degree has a value of x = 3 and $R^1$ is a methyl radical.

Component (B) of the functional fluid composition of general formula (IV) comprises species of ethoxylation degree of from y = 2 to y = 6, preferably of from y = 2 to y = 4. Component (B) may be a single species or a mixture of different species with regard to the ethoxylation degree and/or to radical $R^2$. Radical $R^2$ is preferably a $C_1$- to $C_4$-alkyl radical and may be methyl, ethyl, n-propyl, isopropyl, n-butyl, isobutyl, sec-butyl, tert-butyl, n-pentyl, n-hexyl, n-heptyl, n-octyl and 2-ethylhexyl, ethyl and especially methyl being preferred.

Examples of useful alkoxy glycols for component (B) include methyldiglycol, methyltriglycol, methyltetraglycol, methylpentaglycol, methylhexaglycol, ethyldiglycol, ethyltriglycol, ethyltetraglycol, ethylpentaglycol, ethyl hexaglycol, n-propyl-diglycol, n-propyltriglycol, n-propyltetraglycol, n-propylpentaglycol, n-propylhexaglycol, n-butyldiglycol, n-butyltriglycol, n-butyltetraglycol, n-butylpentaglycol, n-butylhexaglycol, n-pentyldiglycol, n-pentyltriglycol, n-pentyltetraglycol, n-pentylpentaglycol, n-pentyl-hexaglycol, n-hexyldiglycol, n-hexyltriglycol, n-hexyltetraglycol, n-hexylpentaglycol, n-hexylhexaglycol, 2-ethylhexyldiglycol, 2-ethylhexyltriglycol, 2-ethylhexyltetraglycol, 2-ethylhexylpentaglycol, 2-ethylhexylhexaglycol and mixtures thereof. For the avoidance of doubt, "glycol" always means "ethylene glycol".

In a preferred embodiment, component (B) comprises a mixture of alkoxy glycols of general formula (IV) comprising solely or predominantly species with y = 3. Predominantly shall mean that at least 60% by weight, more preferably at least 75% by weight, most preferably at least 90% by weight, of component (B) comprises species with y = 3. In the last case, alkoxy glycol species with y = 2 and/or y = 4 and/or y = 5 and/or y = 6 may be present in minor amounts.

A preferred mixture of such alkoxy glycols for component (B) with y = 3 is a mixture consisting solely or essentially of methyltriglycol and n-butyltriglycol. Typically, the weight ratio of methyltriglycol to n-butyltriglycol in this mixture is from 5 : 1 to 1 : 2, especially from 2 : 1 to 1 : 1.

More preferred are brake fluids in which component (B) is selected from the group consisting of methyltriglycol, methyltetraglycol, n-butyltriglycol, and n-butyltetraglycol.

Preferably the borate esters (A) are selected from the group consisting of borate esters of methyltriglycol, methyltetraglycol, n-butyltriglycol, and n-butyltetraglycol.

According to the invention these alkanol ethoxylates have a low molar share of deuterium:
Preferred methyltriglycol (in formula (II) n=1 and m=3) exhibits a molar share of deuterium of not more than 112.5 ppm, preferably of from 10 to not more than 111, more preferably of from 10 to not more than 109, and even more preferably of from 10 to not more than 105 ppm. These values refer to conventional methanol as starting alkanol for ethoxylation.

In case the methanol is obtained from hydrogenation in step (c) such methyltriglycol exhibits a molar share of deuterium of not more than 100 ppm, preferably of from 10 to not more than 98, more preferably of from 10 to not more than 95, and even more preferably of from 10 to not more than 90 ppm.

Preferred methyltetraglycol (in formula (II) n=1 and m=4) exhibits a molar share of deuterium of not more than 110 ppm, preferably of from 10 to not more than 108, more preferably of from 10 to not more than 106, and even more preferably of from 10 to not more than 102 ppm.

Preferred n-butyltriglycol (in formula (II) n=4 and m=3) exhibits a molar share of deuterium of not more than 123 ppm, preferably of from 10 to not more than 122, more preferably of from 10 to not more than 120, and even more preferably of from 10 to not more than 117 ppm.

Preferred n-butyltetraglycol (in formula (II) n=4 and m=4) exhibits a molar share of deuterium of not more than 119 ppm, preferably of from 10 to not more than 118, more preferably of from 10 to not more than 116, and even more preferably

of from 10 to not more than 113 ppm.

Component (C) of the present functional fluid composition may comprise, besides the alkylamine ethoxylates, at least one additive with corrosion inhibition action, although the alkylamine ethoxylates exhibit corrosion inhibition properties themselves. Suitable customary additives with corrosion inhibition properties include fatty acids such as lauric, palmitic, stearic or oleic acid; esters of phosphorus or phosphoric acid with aliphatic alcohols; phosphites such as ethyl phosphate, dimethyl phosphate, isopropyl phosphate, n-butyl phosphate, triphenyl phosphite and diisopropyl phosphite; heterocyclic nitrogen containing organic compounds such as benzotriazole, tolutriazole, 1,2,4-triazole, benzoimidazole, purine, adenine and derivatives of such heterocyclic organic compounds; alkylamines such as mono- and di-($C_4$- to $C_{20}$-alkyl)amines, e.g. n-butyl-amine, n-hexylamine, n-octylamine, 2-ethylhexylamine, isononylamine, n-decylamine, n-dodecylamine, oleylamine, d-n-proylamine, di-isopropylamine, di-ni-butylamine, di-n-amylamine, cyclohexylamine and salts of such alkylamines; 1- to 5-fold ethoxylated species of the above-mentioned alkylamines such as mono- and di-($C_4$- to $C_{20}$-alkyl)amines, preferably N-hexyl diethanolamine, N-octyl diethanolamine, N-decyl diethanolamine, preferably N-octyl diethanolamine; alkanolamines such as mono-, di- and trimethanolamine, mono-, di- and triethanolamine, mono-, di- and tri-n-propanolamine and mono-, di- and tri-isopropanolamine. Of course, mixtures of the above additives with corrosion inhibition action can be used.

Using alkylamines and/or alkanolamines as compounds with corrosion inhibition action in the additive package of component (C) often results in an additional decrease in viscosity of the present functional fluid composition.

In case ethanolamines are used such ethanolamines may also preferably exhibit a low share of deuterium wherein the molar share of deuterium in each $-CH_2-CH_2-OH$ moiety is not more than 100 ppm, preferably from 10 to 98, more preferably from 10 to 95m and even more preferably from 10 to 90 ppm.

A process for production of such ethanolamines is disclosed in the International Application with the Application No. PCT/EP2023/061271 and the application date 28 April 2023.

Besides the alkylamine ethoxylates and possibly the additives with corrosion inhibition action, further customary additives may be present in the additive package of component (C), for example stabilizers such as pH stabilizers, antioxidants such as phenolthiazine and phenolic compounds, e.g. hydroxyanisol and bisphenol A, defoamers and dyes.

Preferably, the additive package of component (C) includes one or more alkyl-amine ethoxylates consists or consists essentially of a major portion of additives with corrosion inhibition action and a minor portion of additives with antioxidant action and possibly of defoamers and dyes. The portion of alkylamine ethoxylate(s) in the additive package of component (C) is typically of from 1 to 100% by weight, preferably of from 10 to 99% by weight, more preferably of from 25 to 98% by weight, most preferably of from 40 to 97% by weight, each based on the weight of the additive package of component (C).

It is contemplated that also other materials than components (A), (B) and (C) may be formulated into the present functional fluid composition so long as care is taken not to lower the ERBP or WERBP temperatures below the superior high levels of the instant invention or to increase the low temperature viscosity above an acceptable level. For example, the present functional fluid composition may include from 0 to 20% by weight, based on the total weight of the composition, of a diluent or a lubricant such as, for example, polyethylene oxides, polypropylene oxides, poly($C_4$- to $C_{10}$-alkylene) oxides, dialkoxyglycols or borate co-esters.

According to the present invention, the three components (A), (B) and (C) are present in the functional fluid composition in the following amounts:

(A) from 15 to 90% by weight, preferably from 30 to 75% by weight, more preferably from 45 to 65% by weight, most preferably from 56 to 62% by weight;

(B) from 5 to 80% by weight, preferably from 20 to 65% by weight, more preferably from 32 to 52% by weight, most preferably from 36 to 42% by weight;

(C) from 0.1 to 10% by weight, preferably from 0.5 to 7% by weight, more preferably from 1 to 4% by weight, most preferably from 1.5 to 2.5% by weight.

All % values for (A), (B) and (C) above refer to the total composition of the present functional fluid composition, or - if other materials than components (A), (B) and (C), e.g. the above-mentioned diluents and/or lubricants, are present - to the total weight of (A) plus (B) plus (C). The % values for (A), (B) and (C) add up in each case to 100% by weight.

The functional fluid composition of the present invention exhibits superior behavior in ERBP and WERBP temperature and simultaneously in low temperature viscosity performance. Preferably, it exhibits a ERBP of at least 260°C, more preferably of at least 265°C, most preferably of at least 270°C, and/or a WERBP of at least 175°C, more preferably of at least 178°C, still more preferably of at least 180°C, most preferably of at least 182, and especially at least 184 or even 185°C.

The functional fluid composition of the present invention exhibits a low temperature kinematic viscosity of

preferably less than 700 centistokes ("cSt") (= $mm^2/s$), more preferably of less than 685 cSt, most preferably less than 675 cSt, each determined at a temperature of -40°C.

The low viscosity functional fluid composition of the present invention is especially useful as a brake fluid, for example for vehicles such as passenger cars and trucks, especially for new electronic or automated anti-lock brake systems which require lower viscosity fluids for satisfactory operation at low temperatures.

Due to recent developments a demand for borate-free brake fluids has arisen.

Such borate-free brake fluids may comprise

(D) from 50 to 85, preferably 60 - 82 wt.-% of alkoxy glycol according to formula (VI)

H3C-O-(CH2-CH2-O)p-H (VI)

wherein p is a number from 2 to 5, with the proviso that in at least 30 wt.-% of all compounds according to formula (VI) p is 3, and

(E) from 1 to 15, preferably 1.5 to 10 wt.-% of alkoxy glycol according to formula (VII)

R3-O-(CH2-CH2-O)q-H (VII)

wherein

R3 is a C2 to C8 alkyl residue,

q is a number from 2 to 6,

with the proviso that in at least 65 wt.-% of all compounds according to formula (VII) q is 3, and

(F) from 6 to 35, preferably 10 - 25 wt.-% of at least one compound according to formula (VIII)

H-O-(CH2-CH2-O)r-H (VIII)

wherein r is a number of 2 or higher, with the proviso that in at least 80 wt.-% of all compounds according to formula (VIII) r is 2 or 3,

(G) at least one additive, selected from the group consisting of corrosion inhibitor, amines, stabilizers, defoamers and lubricants,

the fluid comprising at most 3 wt.-% of an ester between boric acid and a glycol or polyglycol compound,

wherein components (D), (E), and/or (F) have a low molar share of deuterium according to the present invention, preferably at least two out of components (D), (E), and (F), and very preferably all components components (D), (E), and (F).

Component (D) of the functional fluid according to this invention is a methylterminated polyglycol according to formula (VI). Suitable compounds according to formula (VI) comprise 2, 3, 4 or 5 ethoxy units. Compounds with a higher number of ethoxy units than 5, i.e. 6 ethoxy units or more, may be present but should be restricted to a content of 3 wt.-% at most, relative to the total weight of all compounds according to formula (VI).

The total amount of all compounds according to formula (VI) in the fluid is from 50 to 85 wt.-%, relative to the weight of the fluid, preferably 60 - 82 wt.-%, more preferably 65 - 81 wt.-%, for example preferably 68 - 80 wt.-%. The relative amount of any formula (VI) component with p = 2 preferably is not more than 2.5 wt.-%. The relative amount of any formula (VI) component with p = 3 is at least 30 wt.-%, preferably 50 to 90 wt.-%. The relative amount of any formula (VI) component with p = 4 and 5 is preferably 10- 50 wt.-%, more preferably 13 - 49 wt.-%. All such relative amounts are relative to the total amount of formula (VI) compounds, such total amount being 100 wt.-%. In component (D), species with p = 1 are preferably not present in quantities higher than 1 wt.-%. In component (D), species with p = 6 or higher are preferably not present in quantities higher than 3 wt.-%.

Increasing the amount of formula (VI) components with p = 4 or higher may increase the viscosity of the fluid.

Component (E) of the functional fluid composition, according to formula (VII), comprises species of ethoxylation degrees of from q = 2 to q = 6, preferably of from q = 2 to q = 4. Component (E) may be a single species or a mixture of different species with regards to the ethoxylation degree and/or to radical R3.

Radical R3 is preferably a C2- to C4-alkyl radical. R3 more preferably may be ethyl, n-propyl, isopropyl, n-butyl, isobutyl, sec-butyl, tert-butyl. Ethyl, and especially butyl is most preferred.

Examples of useful alkoxy glycols for component (E) of the present invention include ethyldiglycol, ethyltriglycol, ethyltetraglycol, ethylpentaglycol, ethylhexaglycol, n-propyldiglycol, n-propyltriglycol, n-propyltetraglycol, n-propylpentaglycol, n-propylhexaglycol, n-butyldiglycol, n-butyltriglycol, n-butyltetraglycol, n-butylpentaglycol, n-butylhexaglycol

and mixtures thereof. For the avoidance of doubt, "glycol" always means "ethylene glycol". Preferred are butyltriglycol and butyltetraglycol.

The component (E) preferably comprises a mixture of alkoxy glycols of general formula (VII) comprising solely or predominantly species with q = 3. Predominantly means that at least 65 % by weight, more preferably at least 75 % by weight, of component (E) comprises species with q = 3. Species with q = 4 are preferably present in an amount of 10 wt.-% or more. In any case, alkoxy glycol species with q = 2 and/or q = 5 and/or q = 6 may be present in minor amounts. Species with q = 2 are preferably present in an amount not exceeding 3 wt.-%. Species with q = 5, 6 or higher are preferably present in an amount of less than 5 wt.-% in total.

Weight percentages of species of component (E) are given in wt.-% with the total amount of component (E) being 100 wt.-%. The total amount of all compounds according to formula (VII) in the fluid is from 1 to 15, preferably 1.5 to 10 wt.-%.

Component (F) is a polyethylene glycol according to formula (VIII). In formula (VIII), r is a number of 2, or higher. It is preferred, that r is a number from 2-4. More preferably, r is 2 or 3.

It is required that in at least 80 wt.-% of all compounds according to formula (VIII) r is 2 or 3, the wt.-% being relative to the total weight of all compounds according to formula (VIII). This means that compounds according to formula (VIII) wherein r is 2 or 3 make up 4.8 to 28 wt.-%, preferably 8 to 20 wt.-% of the fluid, the total fluid weight being 100 wt.-%.

In one preferred embodiment, component (F) is a mixture of compounds according to formula (VIII) wherein r is 2 or 3.

The total amount of component (F) in the fluid is from 6 to 35 wt.-%, preferably 10 - 25 wt.-%, more preferably 16 to 23 wt.-% of the weight of the fluid, i.e. the total weight of the fluid being 100 wt.-%.

In one preferred embodiment, the amount of species of formula (VIII) wherein r = 2 is 6 - 12 wt.-%. In one other preferred embodiment, the amount of species of formula (VIII) wherein r = 3 is 5 - 15 wt.-%. In one other preferred embodiment, the total amount of species according to formula (VIII) wherein r is 4 or higher than 4 is at most 8 wt.-%, more preferably at most 5 wt.-%. Said weight percentages provided for species according to formula (VIII) are provided as weight percentages of the fluid, i.e. the total weight of the fluid is 100 wt.-%. They are not provided as weight percentages of the total weight of component (F).

Component (G) is an additive that is required to impart particular properties to the functional fluid for performing on specifications to be fulfilled for brake fluids according to the current norms and standards FMVSS No. 116, SAE J 1703 and ISO 4925.

Such components (G) encompass the individuals disclosed above as components (C) for borate ester-containing brake fluids.

The preferred total amount of all components (G) in the fluid is from 0.4 to 6 wt.-%, more preferably from 0.5 to 5.5 wt.-%.

Component (G) comprises one or more additives selected from the group consisting of corrosion inhibitors, amines as reserve alkalinity agents, stabilizing antioxidants, defoamers, lubricants and dyes.

In one preferred embodiment, the % values (A) - (G) add up to 100% by weight.

The total content of boric acid esters in the fluid is at most 3 wt.-%, preferably less than 0.3 wt.-%.

In one preferred embodiment, the weight ratio between component (D) and component (E) is (D):(E) = 82: 1 to 6: 1.

In one preferred embodiment, the weight ratio between compounds according formula (VI) with p = 3 and p = 4 or higher is (p = 3):(p = 4, or higher) = 1 : 1 to 6 : 1.

In one preferred embodiment, the weight ratio between component (A) and component (F) is (D):(F) = 2.0 : 1 to 8.2 : 1.

In one embodiment, the subject matter of this invention corresponds to a functional fluid, comprising

(D) from 50 to 85, preferably 60 - 82 wt.-% of alkoxy glycol according to formula (VI)

$$H3C\text{-}O\text{-}(CH2\text{-}CH2\text{-}O)p\text{-}H \qquad (VI)$$

wherein p is a number from 2 to 5, with the proviso that in at least 30 wt.-% of all compounds according to formula (VI) p is 3, and

(E) from 1 to 15, preferably 1.5 to 10 wt.-% of alkoxy glycol according to formula (VII)

$$R3\text{-}O\text{-}(CH2\text{-}CH2\text{-}O)q\text{-}H \qquad (VII)$$

wherein
R3 is a C2 to C8 alkyl residue,
q is a number from 2 to 6,

with the proviso that in at least 65 wt.-% of all compounds according to formula (VII) q is 3, and
(F) from 6 to 35, preferably 10 - 25 wt.-% of at least one compound according to formula (VIII)

H-O-(CH2-CH2-O)r-H (VIII)

wherein r is a number of 2 or higher, with the proviso that in at least 80 wt.-% of all compounds according to formula (III) r is 2 or 3,
(G) at least one additive, selected from the group consisting of corrosion inhibitor, amines, stabilizers, defoamers and lubricants,
the fluid comprising at most 3 wt.-% of an ester between boric acid and a glycol or alkyl polyglycol compound,
wherein component (G) is present in an amount of 0.4 to 6 wt.-%, preferably 0.5 to 5.5 wt.-%, and
wherein the corrosion inhibitor is selected from the group consisting of C8 to C22 fatty acids, esters of phosphorus or phosphoric acid with C1 to C18 aliphatic alcohols, phosphites having at least one C1 to C12 hydrocarbon residue; heterocyclic organic compounds having at least one nitrogen atom as heteroatom, and mixtures thereof; and
wherein the amine is selected from the group consisting of alkyl or cycloalkyl amines, alkanol amines, alkyl amine ethoxylates and their mixtures; and
wherein the stabilizer is selected from the group consisting of substituted phenols, sterically hindered amines and mixtures thereof; and
wherein the defoamer is selected from the group consisting of natural oils, glycerides, waxes, fine powdered silica, ethylene oxide/propylene oxide block copolymers, silicone based defoam and mixtures thereof; and
wherein the lubricant is selected from the group consisting of polypropylene oxides, random poly (C2- to C4-alkylene) oxides, random mono C1 to C4 substituted poly (C2- to C4-alkylene) oxides, castor oil, ricinoleic acid, ethoxylates of castor oil or ricinoleic acid, and mixtures thereof.

Further borate-free brake fluids are described in the International Application with the Application No. PCT/EP2023/063884 filed 24 May 2023 using silicon-containing component (J) rather than esters of boric acid:

Such silicon-containing brake fluids may comprise
(D) at least one alkoxy glycol according to formula (VI)

H3C-O-(CH2-CH2-O)p-H (VI)

wherein p is a number from 2 to 5, with the proviso that in at least 30 wt.-% of all compounds according to formula (VI) p is 3, and
(E) at least one alkoxy glycol according to formula (VII)

R3-O-(CH2-CH2-O)q-H (VII)

wherein
R3 is a C2 to C8 alkyl residue,
q is a number from 2 to 6,
with the proviso that in at least 65 wt.-% of all compounds according to formula (VII) q is 3, and
(F) optionally at least one compound according to formula (VIII)

H-O-(CH2-CH2-O)r-H (VIII)

wherein r is a number of 2 or higher, with the proviso that in at least 80 wt.-% of all compounds according to formula (VIII) r is 2 or 3,
(G) at least one additive, selected from the group consisting of corrosion inhibitor, amines, stabilizers, defoamers and lubricants,
(J) at least one organic silicon compound of formula (IX)

R4s-Si(-[-O-CH2-CH2-]t-O-R5)4-s

wherein

s is a positive integer 1, 2 or 3, preferably 1 or 2, more preferably 2,
t is a positive integer from 2 to 4, preferably 3 or 4, more preferably 3,
R4 C1- to C4-alkyl, preferably methyl or ethyl, more preferably methyl and
R5 C1- to C4-alkyl, preferably methyl or n-butyl, more preferably methyl
comprising
(D) from 24 to 45 wt%, preferably 24 to 40, more preferably 24 to 35, and especially 24 to 30 wr%,
(E) from 5 to 20, preferably 7 to 15 wt%,
(F) 0 to 10, preferably 0 to 5 wt%,
(G) more than 0 to 5 wt%, preferably 1 to 4 wt%, and
(J) from 40 to 55, preferably 43 to 55, even more preferably 45 to 53, and especially 47 to 52 wt%,
with the proviso that the components (D) to (G) and (J) always add up to 100 wt%,
wherein components (D), (E), (F) and/or (J) have a low molar share of deuterium according to the present invention, preferably at least two out of components (D), (E), (F) and (J), very preferably at least three out of components components (D), (E), (F) and (J), and especially all four components (D), (E), (F) and (J).

Components (D) to (G) are the same compounds as described above for the borate-free brake fluids.
The total content of boric acid esters in the fluid is at most 3 wt.-%, preferably less than 0.3 wt.-%.
In preferred silicon-containing brake fluids

- the sum of all glycols and alkoxy glycols with p, q, r, and t = 2 is more than 0 (zero) and not more than 10 mol%,
- the sum of all glycols and alkoxy glycols with p, q, r, and t = 3 is at least 60 mol%, and
- the sum of all glycols and alkoxy glycols with p, q, r, and t = 4 is more than 0 (zero) and not more than 20 mol%.

In the compounds (J), the substituent R4 can be C1 to C4 alkyl, preferably methyl, ethyl or n-butyl, especially methyl or ethyl and especially methyl. The s substituents R4 can be independently of each other the same or different, preferably they are the same.
The integer s of substituent R4 is from 1 to 3, preferably 1 or 2 and especially preferably 2.
The substructures H-[-O-CH2-CH2-]t-O-R5 are alkylene glycol monoalkyl-ether, as described above for compounds (D) and (E), R5 is C1- to C4-alkyl, preferably methyl, ethyl or n-butyl, very preferably methyl or n-butyl, and methyl is particularly preferred. The running index t is a positive integer from 2 to 4, preferably 3 or 4 and especially preferably 3.
In a preferred embodiment, the substructures H-[-O-CH2-CH2-]t-O-R5 are selected from the group consisting of diethylene glycol monomethyl ether, triethylene glycol mono-methyl ether, tetraethylene glycol monomethyl ether, diethylene glycol monoethyl ether, triethylene glycol monoethyl ether, tetraethylene glycol monoethyl ether, diethylene glycol mono n-butyl ether, triethylene glycol mono n-butyl ether and tetraethylene glycol mono n-butyl ether, preferably triethylene glycol monomethyl ether, tetraethylene glycol monomethyl ether, triethylene glycol mono n-butyl ether and tetraethylene glycol mono n-butyl ether, triethylene glycol monomethyl ether and triethylene glycol mono n-butyl ether are particularly preferred.
Examples of useful components (J) include those in which in formula (IX)

- R4 is methyl, s = 2, and (H-[-O-CH2-CH2-]t-O-R5) is methyl triethylene glycol,
- R4 is methyl, s = 2, and (H-[-O-CH2-CH2-]t-O-R5) is ethyl triethylene glycol,
- R4 is methyl, s = 2, and (H-[-O-CH2-CH2-]t-O-R5) is n-butyl triethylene glycol,
- R4 is methyl, s = 2, and (H-[-O-CH2-CH2-]t-O-R5) is methyl tetraethylene glycol,
- R4 is methyl, s = 2, and (H-[-O-CH2-CH2-]t-O-R5) is ethyl tetraethylene glycol,
- R4 is methyl, s = 2, and (H-[-O-CH2-CH2-]t-O-R5) is n-butyl tetraethylene glycol,
- R4 is ethyl, s = 2, and (H-[-O-CH2-CH2-]t-O-R5) is methyl triethylene glycol,
- R4 is ethyl, s = 2, and (H-[-O-CH2-CH2-]t-O-R5) is ethyl triethylene glycol,
- R4 is ethyl, s = 2, and (H-[-O-CH2-CH2-]t-O-R5) is n-butyl triethylene glycol,
- R4 is ethyl, s = 2, and (H-[-O-CH2-CH2-]t-O-R5) is methyl tetraethylene glycol,
- R4 is ethyl, s = 2, and (H-[-O-CH2-CH2-]t-O-R5) is ethyl tetraethylene glycol,
- R4 is ethyl, s = 2, and (H-[-O-CH2-CH2-]t-O-R5) is n-butyl tetraethylene glycol,

In a preferred embodiment, the substructures H-[-O-CH2-CH2-]t-O-R5 are the same alkylene glycol monoalkyl ethers as they are used as components (D) and/or (E), especially preferably the same alkylene glycol monoalkyl ethers as they are used as components (D).
The coolants and brake fluids described herein are usually manufactured by blending and mixing the components with each other. This may take place in vessels (stirred tank reactors, circular pumping, inert gas injection for agitation), tanks (nozzles, injectors, gas induced mixing), inline blending (potentially assisted by static mixers) and

optionally direct loading into vessels, tanks, packaging (IBCs, drums or barrels) or transport vehicles (e.g. railcars, trucks).

Further uses

The ethylene glycols and/or alkoxylated compounds of the present invention having a low molar share of deuterium have a wide range of applications across various industries. Some of the applications are:

- Lubricants: The ethylene glycols and/or alkoxylated compounds can be used as lubricants in various industries such as automotive, aerospace, and industrial machinery. They offer excellent lubrication properties, high thermal stability, and resistance to oxidation.
- Personal care products: The ethylene glycols and/or alkoxylated compounds can be used in formulations of personal care products such as lotions, creams, and shampoos. They provide for example moisturizing and conditioning properties to the skin and hair.
- Home care products: The ethylene glycols and/or alkoxylated products can be used as surfactants in laundry detergents, hard surface cleaner, and rinse aids. They provide excellent wetting, cleaning, and emulsifying properties
- Pharmaceutical industry: The ethylene glycols and/or alkoxylated compounds can be used as excipients in the pharmaceutical industry to improve drug solubility, stability, and bioavailability. They are also used in formulations of ointments, creams, and gels.
- Textile industry: The ethylene glycols and/or alkoxylated compounds can be used in the textile industry as softeners, moisture- and anti-static agents. They can improve the texture and feel of fabrics and reduce static electricity.
- Food industry: The ethylene glycols and/or alkoxylated compounds can be used in the food industry as emulsifiers, thickeners, and stabilizers. They can be used in the production of ice cream, dairy products, and baked goods.
- Oil and gas industry: The ethylene glycols and/or alkoxylated compounds can be used as hydraulic fluids, O/W or W/O emulsion breakers, foamers, gas hydrate inhibitors, dispersants for inorganic or organic deposits and heat transfer fluids in the oil and gas industry. Furthermore, oligomeric ethylene glycols and/or alkoxylated compounds play an important role in enhanced oil recovery (tertiary oil recovery) for increasing the yield of oilfield exploitation. They offer for example excellent wetting and lubrication properties and high thermal stability.
- Agriculture/Agrochemicals: The ethylene glycols and/or alkoxylated compounds can be used as adjuvants in the agriculture industry for example to improve the effectiveness of herbicides and pesticides.
- Chemical industry: The ethylene glycols and/or alkoxylated compounds can be used as reaction media, surfactants, and dispersants in the chemical industry. They can be used in the production of or as solvent for polymers, resins, adhesives, and coatings.
- Building materials: The ethylene glycols and/or alkoxylated compounds can be used in the construction industry as additives in cement, concrete, and plaster to improve for example their workability, strength, and durability.
- Polyurethane production: The ethylene glycols and/or alkoxylated compounds can be used as starting materials for the production of for example polyurethane foams, adhesives, and coatings. They can act as chain extenders and cross-linking agents in the polymerization process.
- Metalworking fluids: The ethylene glycols and/or alkoxylated compounds can be as coolants and lubricants in metalworking processes such as cutting, grinding, and drilling. They offer for example excellent thermal stability, low volatility, and high lubricity.
- Electronics: The ethylene glycols and/or alkoxylated compounds can be used as heat transfer fluids in electronic cooling systems. They offer for example high thermal conductivity, low viscosity, and compatibility with various materials.
- Fuel and energy: The ethylene glycols and/or alkoxylated compounds can be used as additives in fuels and lubricants, for example fuel performance packages, to improve their performance and reduce emissions. They can also be used as heat transfer fluids in solar and geothermal energy systems. Furthermore, they can be used for absorbing gaseous water from natural or refinery gas or circular gas in hydroraffination.
- Traffic: The ethylene glycols and/or alkoxylated compounds can be used in de-icing liquids for planes, landing strips or roads
- Water treatment: The ethylene glycols and/or alkoxylated compounds can be used as flocculants and coagulants in water treatment processes. They can help removing suspended particles and impurities from water.
- Adhesives and sealants: The ethylene glycols and/or alkoxylated compounds can be used as binders and thickeners in the formulation of adhesives and sealants. They can provide improved adhesion, flexibility, and moisture resistance, e.g. for packaging purposes using e.g. polyethylene terephthalate.

The present invention therefore further relates to the use of the inventive ethylene glycols and/or alkoxylated compounds in any one of the applications mentioned above.

Preferably, the present invention therefore further relates to the use of the ethylene glycols and/or alkoxylated

compounds according to the present invention or obtained by a process according to the present invention in home care products, cosmetic products, pharmaceutical products, food sector, building materials, lubricants like engine oils, bearing oils, gear oils, compressor oils, lubricating greases, heat transfer fluids, metalworking fluids and transmission fluids, antifoaming agents, softeners, rheology modifiers, emulsifiers, dispersing agents, thickeners, stabilizers, metal working fluids, agrochemicals like pesticides, textile and leather auxiliaries, bioprocessing, fuel performance packages and poly(urethane) applications, and home care products, cosmetic products, pharmaceutical products, products in food sector, building materials, lubricants like engine oils, bearing oils, gear oils, compressor oils, lubricating greases, heat transfer fluids, metalworking fluids and transmission fluids, antifoaming agents, softeners, rheology modifiers, emulsifiers, dispersing agents, thickeners, stabilizers, metal working fluids, agrochemicals like pesticides, textile and leather auxiliaries, bioprocessing, fuel performance packages and poly(urethane) applications comprising at least one the ethylene glycols and/or alkoxylated compound according to the present invention or obtained by a process according to the present invention.

Tracing

The ethylene glycols, selected from mono ethylene glycol, oligo ethylene glycols, poly ethylene glycols, and mixtures thereof, and alkanol ethoxylates according to the present invention are characterized by a low deuterium molar share.

They display a deuterium share different from petrochemically made polymers and ethylene glycols, selected from mono ethylene glycol, oligo ethylene glycols, poly ethylene glycols, and
mixtures thereof, and alkanol ethoxylates made by a petrochemical process, i.e. based on fossil energy.

Said low deuterium molar share is mainly introduced by step (a) of the process according to the present invention. Therefore, also the methanol, ethylene, and ethylene oxide, prepared by reacting carbon oxides with the hydrogen from step (a) is characterized by a low deuterium molar share.

With the present invention environmentally friendly ethylene glycols, selected from mono ethylene glycol, oligo ethylene glycols, poly ethylene glycols, and mixtures thereof, and alkanol ethoxylates and an environmentally friendly process for making the same, wherein said process uses as little fossil energy as possible, are provided.

It has been found that said environmentally friendly prepared compounds, i.e. ethylene glycols, selected from mono ethylene glycol, oligo ethylene glycols, poly ethylene glycols, and mixtures thereof, and alkanol ethoxylates are characterized by a specifically low deuterium molar share.

As mentioned above, it is important that the origin of the hydrogen and downstream compounds obtained by clean energy can be tracked in a reliable way.

Today, the majority of hydrogen is produced from fossil fuels by steam reforming of natural gas and other light hydrocarbons, partial oxidation of heavier hydrocarbons, and coal gasification.

However, up to now, there was no possibility to distinguish the hydrogen obtained by by steam reforming, partial oxidation and coal gasification, i.e. by fossil resources, from hydrogen obtained by electrolysis. As discussed above, hydrogen obtained by electrolysis is preferably obtained by using non-fossil energy sources. It is expected that the electrification (power generation) of fossil sources will be fully replaced by the generation of power by non-fossil resources in the near future.

The inventors therefore found a way for tracing the origin of hydrogen and downstream products of hydrogen, here ethylene glycols, selected from mono ethylene glycol, oligo ethylene glycols, poly ethylene glycols, and mixtures thereof, and alkanol ethoxylates via the deuterium molar share of said compounds. The downstream products based on hydrogen here ethylene glycols, selected from mono ethylene glycol, oligo ethylene glycols, poly ethylene glycols, and mixtures thereof, and alkanol ethoxylates based on hydrogen obtained by electrolysis and hydrogen itself can be distinguished by its deuterium molar share from here ethylene glycols, selected from mono ethylene glycol, oligo ethylene glycols, poly ethylene glycols, and mixtures thereof, and alkanol ethoxylates prepared by processes based on fossil energy, i.e. made by petrochemical processes.

Furthermore by using carbon oxides such as carbon monoxide and preferably carbon dioxide together with hydrogen instead of petrochemical synthesis gas in the subsequent synthesis routes for here ethylene glycols, selected from mono ethylene glycol, oligo ethylene glycols, poly ethylene glycols, and mixtures thereof, and alkanol ethoxylates the molar share of deuterium in these compounds was found to be uniquely low with excellent traceability.

The present invention therefore relates to the use of the molar share of deuterium in hydrogen and downstream compounds based on hydrogen for tracing the origin, especially the energetic origin, of the hydrogen and downstream compounds based on hydrogen, wherein the compounds are here ethylene glycols, selected from mono ethylene glycol, oligo ethylene glycols, poly ethylene glycols, and mixtures thereof, and alkanol ethoxylates.

The present invention further relates to a process for tracing the origin, especially the energetic origin, of hydrogen and downstream compounds based on hydrogen by determining the molar share of deuterium in hydrogen and said downstream compounds based on hydrogen, wherein the compounds are here ethylene glycols, selected from mono

ethylene glycol, oligo ethylene glycols, poly ethylene glycols, and mixtures thereof, and alkanol ethoxylates.

Downstream products based on hydrogen are generally products prepared by using hydrogen, here ethylene glycols, selected from mono ethylene glycol, oligo ethylene glycols, poly ethylene glycols, and mixtures thereof, and alkanol ethoxylates as well as methanol. The preparation of the downstream products as well as other downstream products based on hydrogen are known in the art.

Tracing is in the meaning of the present invention synonymous with tracking.

The origin is in the meaning of the present invention the preparation method of the hydrogen employed, especially electrolysis and/or the energetic origin, i.e. non-fossil energy sources. As mentioned above, it is expected that the electrification (power generation) of fossil sources will be fully replaced by the generation of power by non-fossil resources in the near future. Hydrogen made by electrolysis is in this case hydrogen of non-fossil origin. Examples for non-fossil power sources are mentioned above.

The inventive process for tracing the origin, especially the energetic origin, of hydrogen and downstream compounds mentioned above may be employed as a single tracing (tracking) method or in combination with further tracing (tracking) methods.

Suitable deuterium molar shares of the compounds based on hydrogen, here ethylene glycols, selected from mono ethylene glycol, oligo ethylene glycols, poly ethylene glycols, and mixtures thereof, and alkanol ethoxylates, and the hydrogen itself obtained based on hydrogen made by electrolysis, especially obtained by the process of the present invention, are mentioned in the present application.

The invention is further illustrated by working examples.

Experimental Results - Overview

Table 1

| Electrolyser type | Example I) PEM-electrolyser | Example II) PEM electrolyser | Example III) Alkaline electrolyser | Example IV) Alkaline electrolyser | Example V) AEM | Example VI) AEM | Example VII) Solid oxide |
|---|---|---|---|---|---|---|---|
| Water-type/deuterium content | VSMOW/155,76 ppm D | Selected* surface water/139 ppm D | VSMOV/155,76 ppm D | Selected* surface water/139ppm D | VSMOW/155,76 ppm D | Selected* surface water/139 ppm D | Selected* surface water/139 ppm D |
| Current density [A/cm2] | 1.35 | 1.35 | 0.8 | 0.8 | 1.6 | 1.6 | 0.78 |
| Applied voltage [V] | 1.9 | 1.9 | 2.15 | 2.15 | 1.9 | 1.9 | 1.35 |
| Operating temperature [°C] | 68 | 68 | 81 | 81 | 45 | 45 | 710 |
| Cell pressure [bar] | 23 | 23 | 25 | 25 | 35 | 35 | 1.3 |
| Voltage efficiency [%] | 57 | 57 | 53 | 53 | 66 | 66 | 81 |
| Electrical efficiency [kWh/kg H2] | 52 | 52 | 48.5 | 48.6 | 69 | 69 | 47 |
| Deuterium content in hydrogen [ppm] | 86 | 77 | 95 | 87 | 74 | 66 | 38 |

* Isar river surface water (Munich) collected in winter season January 2021.

I Hydrogen Production

Experimental Setup and Method: Electrolysis cell design and Hydrogen production conditions

1) Polymer Electrolyte Membrane / Proton Exchange Membrane Electrolysis (PEM) Electrolysis Cell

Water electrolysis was conducted with a circular commercial PEM electrolysis cell (model ZE 200, Sylatech Analysetechnik GmbH, 0.007 $m^2$ active area). The cell stack was sealed with O-rings and wrapped with heat insulation fabric for isothermal operating conditions. A Nafion® 117 standard membrane (supplier DuPont, dry thickness 180 microns) was assembled by HIAT GmbH with catalytic active coating materials iridium (19 $g/m^2$) and platinum (8 $g/m^2$). Water distribution at the anode half-cell was realized with a titanium mesh. Before the polymer electrolyte membrane, a porous transport layer with sintered titanium fiber material is used for controlled flow while a porous graphite plate was situated on the cathode-side.

Experimental Conditions

Water with controlled temperature was supplied at constant flow of 9.5 g/h to the anode compartment. The cell pressure on cathode and anode side was controlled with PC valves. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank Anodic cell-water was re-cycled, whereas on the cathode the separated water was drained. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

2) Alkaline Electrolysis Cell (AEC)

Electrolysis Cell

Alkaline water electrolysis was conducted with a circular AEC electrolysis cell (model Electro MP Cell, supplier ElectroCell Europe A/S, 0.01 $m^2$ electrode area). As electrodes Nickel 2.4068 material was used and separated by a commercial standard Zirfon Perl UTP 500 membrane (open mesh polyphenylene sulfide fabric symmetrically coated with a mixture polymer / zirconium oxide; thickness 500 microns; 0.023 $m^2$ active area; supplier Agfa-Gevaert N.V.) in zero-gap cell configuration.

Experimental Conditions

Alkaline water (32 wt% potassium hydroxide, technical standard grade) with controlled temperature was supplied at constant flow of 27.8 kg/h to the anode and cathode compartment. The cell pressure on cathode and anode side was equalized via PC valve control. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank Anodic and cathodic cell-water was re-cycled. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

3) Anion Exchange Membrane / Alkaline Electrolyte Membrane Electrolysis (AEM)

Electrolysis Cell

The AEM experiments were executed in a commercial, fully automated 2.4 kW EL 4.0 cell supplied by Enapter GmbH, 10117 Berlin and a 1 wt% potassium hydroxide (standard grade) electrolyte solution.

Test station

As recommended by the supplier the EL 4.0 electrolyzer is run with a 1 wt% potassium hydroxide (technical standard grade) solution, hydrogen production at operating conditions (experimental conditions such as temperature and pressure settings see table) was 480 l/h at approx. 400 ml water consumption. The evolved hydrogen gas was treated with

desiccant dryers, ensuring complete separation of moisture in the electrolysis gas flow to yield < 0,03 wt% water in the hydrogen gas stream. Small gas samples of the dehydrated cathodic hydrogen gas were taken from the continuous flow with an auto-sampler at regular intervals to conduct hydrogen gas analytics described below.

4) Solid Oxide Electrolysis Cell (SOE)

Solid Oxide cell stacks from Elcogen- Elco Stack (Elcogen OY, Vantaa 01510 Finland), were used and a E3000 unit was operated in reverse mode at 700°C and 35A electrolyzing current; Anode functional composition due to the supplier is NiO/YSZ. Cathode is of LSC type [La(Sr)CoO3]: The principle is also described in Novel high-performance solid oxide fuel cells with bulk ionic conductance dominated thin-film electrolytes - ScienceDirect; D. Stöver et al, Journal of Power Sources; Volume 218, 15 November 2012, Pages 157-162.

The hydrogen stream was used with no further purification/dryer. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

The results of the hydrogen production are shown in table 1.

Experimental Setup and Method: "D content (Deuterium content) in samples"

The following method descriptions apply for the determination of the molar share of deuterium based on the total hydrogen content (Deuterium content) of gas and liquid samples. The isotopic H/D-share analysis is based on mass spectroscopy. Two different methods are used: method A for gas samples and method B for liquid samples.

For determination of the "D content in gas and liquid samples" it is of crucial importance not to contaminate the samples e.g. with ambient humidity or other ambient components containing hydrogen or deuterium. Therefore gas-tight materials and sealings must be used with clean sample containers to avoid any cross-contamination. Therefore, before filling and sealing a sample container it must be flushed at least 20 times the sample container volume with the gas or liquid stream to be analyzed. The same is valid for the experimental setup of the gas sampler and mass spectrometer. Utmost care must be taken to avoid cross-contamination e.g. via condensation of humidity. The analytical setup from sampling to mass spectrometry is validated with known reference samples.

Method A) Gas samples

Total Deuterium from HD and $D_2$ in hydrogen gas samples was determined via ultra-high resolution quadrapole mass spectrometry using a Hiden DLS-20 (Hiden Analytical Ltd., Warrington, Cheshire, UK) analyzer setup. The general method setup is described in C.C. Klepper, T.M. Biewer, U. Kruezi, S. Vartanian, D. Douai, D.L. Hillis, C. Marcus, Extending helium partial pressure measurement technology to JET DTE2 and ITER; Rev. Sci. Instrum., 87 (11) (2016); doi: 10.1063/1.4963713. For the hydrogen gas samples the threshold ionization mass spectrometry mode (TIMS) was used as described in S. Davies, J.A. Rees, D.L. Seymour; Threshold ionisation mass spectrometry (TIMS); A complementary quantitative technique to conventional mass resolved mass spectrometry; Vacuum, 101 (2014), pp. 416-422; doi: 10.1016/j.vacuum.2013.06.004. Sensitivity is +/-1 ppm.

Method B) Liquid samples

Analysis of liquid samples was executed via isotope ratio monitoring gas chromatography/mass spectrometry (IRMS). Therefore a DELTA V PLUS CF-IRMS mass spectrometer was used. This mass spectrometer with magnetic sector with continuous flux DELTA V PLUS CF-IRMS is used to measure the isotopic ratio of 2 H/1 H.

Measurement of D/H in a continuous He-flow mode needs the complete removal of low energy 4 He+ ions from the HD+ ion beam at m/z 3). The method is described in RAPID COMMUNICATIONS IN MASS SPECTROMETRY Rapid Commun. Mass Spectrom. 13, 1226-1230 (1999), W.A. Brandt et al. Sensitivity is within +/-3 ppm.

II Production of mono ethylene glycol and tri ethylene glycol mono methyl ether

In table 2, the deuterium (D) content in hydrogen, mono ethylene glycol (MEG), and tri ethylene glycol mono methyl ether (MTG, methyl triglycol) based on fossil resources (FR's) (comparative examples) and non-fossil resources (NFR's) (inventive examples) is shown.

The deuterium contents of products from FR's (comparative examples) are assumed for conventional petrochemical products to be 150 ppm.

The deuterium contents of products from NFR's are calculated as described above using the D content of hydrogen from examples I) to VI) (see table 1) as starting materials for the ethylene synthesis routes.

| | Comparative examples: Products based on fossil resources (FR) | | |
|---|---|---|---|
| Comparative Example | | D content [ppm] in MEG from FR's | D content [ppm] in MTG from FR's |
| | | 150 | 150 |
| | | | |
| Inventive Examples | D content [ppm] in hydrogen from NFR's | D content [ppm] in MEG from NFR's (*) | D content [ppm] in MTG from NFR's (**) |
| AI) | 86 | 107 | 102 |
| AII) | 77 | 101 | 95 |
| AIII) | 95 | 113 | 109 |
| AIV) | 87 | 108 | 103 |
| AV) | 74 | 99 | 93 |
| AVI) | 66 | 94 | 87 |
| AVII) | 38 | 75 | 66 |
| (*) Using conventional water with a deuterium content of 150 ppm as starter<br>(**) Using conventional methanol with a deuterium content of 150 ppm as starter | | | |

The examples based on NFR's show a significantly lower D content and can be clearly distinguished from conventional products based on FR's.

**Claims**

1. Process for making ethylene glycols, selected from mono ethylene glycol, oligo ethylene glycols, poly ethylene glycols, and mixtures thereof, and alkanol ethoxylates, wherein said process comprises the following steps:

   (a) providing hydrogen with a molar share of deuterium ≤ 100 ppm, based on the total hydrogen content, by electrolysis based on electrical power generated at least in part from non-fossil energy,
   (b) providing carbon oxides, preferably carbon dioxide,
   (c) reacting the hydrogen from step (a) with carbon oxides, preferably carbon dioxide from step (b) to form methanol,
   (d) converting the methanol from step (c) to ethylene and further to ethylene oxide,
   (e) converting water resp. alkanols $C_nH_{2n+1}$-OH with ethylene oxide from step (d) to ethylene glycols resp. alkanol ethoxylates in one or more steps.

2. The process according to claim 1 wherein the electrical power is generated at least in part from wind power, solar energy (thermal, photovoltaic and concentrated), hydroelectricity (tidal power, wave power, hydroelectric dams, In-river-hydrokinetics), geothermal energy, ambient heat captured by heat pumps, bioen-ergy (biofuel, biomass), the renewable part of waste or nuclear power (fission, fusion).

3. The process according to claim 1 or 2, wherein step (a) is a water electrolysis, preferably PEM water electrolysis, alkaline water electrolysis, or AEM water electrolysis.

4. The process according to any one of claims 1 to 3, wherein carbon dioxide is employed in step (c), which is preferably captured from industrial flue gases or from ambient air.

5. The process according to any one of claims 1 to 4 wherein the ethylene oxide in step (d) is obtained by

   (d1) a methanol-to-olefin process, preferably with a zeolite catalyst, wherein ethylene is obtained, followed by
   (d2) epoxidation of ethylene, preferably with a silver-based catalyst.

6. Ethylene glycols of the general formula

(I) $HO-[-CH_2-CH_2-O-]_m-H$

wherein

m is a positive integer from 1 to 100,
wherein the molar share of deuterium is
not more than

$$[(2 \times 150) + (4 \times m \times 100)] / (2 + (4 \times m)) \text{ ppm},$$

preferably in the range of
from

$$[(2 \times 150) + (4 \times m \times 10)] / (2 + (4 \times m))$$

to

$$[(2 \times 150) + (4 \times m \times 98)] / (2 + (4 \times m)) \text{ ppm},$$

more preferably
from

$$[(2 \times 150) + (4 \times m \times 10)] / (2 + (4 \times m))$$

to

$$[(2 \times 150) + (4 \times m \times 95)] / (2 + (4 \times m)) \text{ ppm},$$

most preferably
from

$$[(2 \times 150) + (4 \times m \times 10)] / (2 + (4 \times m))$$

to

$$[(2 \times 150) + (4 \times m \times 90)] / (2 + (4 \times m)) \text{ ppm},$$

based on the total hydrogen content.

**7.** Use of ethylene glycols according to Claim 6 in coolants, brake fluids, cosmetics, shampoos, soaps, nonionic or ionic detergents, personal hygiene applications, and polymers, such as poly ethylene terephthalate.

**8.** Alkanol ethoxylates are of the general formula

(II) $H_{2n+1}C_n-O-[-CH_2-CH_2-O-]_m-H$

wherein

n is a positive integer from 1 to 20, and
m is a positive integer from 1 to 100
wherein the molar share of deuterium is
not more than $[(((2 \times n) + 2) \times 150) + (4 \times m \times 100)] / (((2 \times n) + 2) + (4 \times m))$ ppm,
preferably in the range of

from

$$[(((2 \times n) + 2) \times 150) + (4 \times m \times 10)] / (((2 \times n) + 2) + (4 \times m))$$

to

$$[(((2 \times n) + 2) \times 150) + (4 \times m \times 98)] / (((2 \times n) + 2) + (4 \times m)) \text{ ppm,}$$

more preferably in the range of
from

$$[(((2 \times n) + 2) \times 150) + (4 \times m \times 10)] / (((2 \times n) + 2) + (4 \times m))$$

to

$$[(((2 \times n) + 2) \times 150) + (4 \times m \times 95)] / (((2 \times n) + 2) + (4 \times m)) \text{ ppm}$$

most preferably in the range of
from

$$[(((2 \times n) + 2) \times 150) + (4 \times m \times 10)] / (((2 \times n) + 2) + (4 \times m))$$

to

$$[(((2 \times n) + 2) \times 150) + (4 \times m \times 90)] / (((2 \times n) + 2) + (4 \times m)) \text{ ppm,}$$

based on the total hydrogen content.

**9.** Use of alkanol ethoxylates according to Claim 8 in coolants, brake fluids, cosmetics, shampoos, soaps, nonionic or ionic detergents, and personal hygiene applications.

**10.** Use of the molar share of deuterium in hydrogen and downstream compounds based on hydrogen for tracing the origin, especially the energetic origin, of the hydrogen and downstream compounds based on hydrogen, wherein the compounds are ethylene glycols, selected from mono ethylene glycol, oligo ethylene glycols, poly ethylene glycols, and mixtures thereof, and alkanol ethoxylates.

**11.** A process for tracing the origin, especially the energetic origin, of hydrogen and downstream compounds based on hydrogen by determining the molar share of deuterium in in hydrogen and said downstream compounds based on hydrogen, wherein the compounds are ethylene glycols, selected from mono ethylene glycol, oligo ethylene glycols, poly ethylene glycols, and mixtures thereof, and alkanol ethoxylates.

**12.** Coolants, comprising

(A) at least one ethylene glycol according to Claim 6.
(B) water
(C) at least one azole derivative
(D) optionally at least one ester of orthosilicic acid or alkoxy alkylsilane
(E) optionally at least one inorganic salt selected from the group consisting of molybdates, borates, phosphates, silicates, nitrites and nitrates,
(F) optionally at least one mono- or dicarboxylic acid
(G) optionally at least one silicophosphonate
(H) optionally at least one tertiary amine
(I) optionally at least one further coolant additive,

wherein

at least one inorganic salt and/or at least one carboxylic acid is present.

**13.** Use of coolants according to Claim 12 in cooling systems of stationary engines or vehicles with combustion engines, electric engines, fuel cells or hybrid engines with a combination of combustion engines with electric engines or a combination of combustion engines with fuel cells, or in generators, such as wind turbines.

**14.** Borate-containing brake fluids comprising

(A) from 15 to 90% by weight, based on the weight of the total composition, of one or more alkoxy glycol borate esters having the general formula (III)

$$[R^1\text{-O-}(CH_2CH_2\text{-O})_x]_3B \qquad \text{(III)}$$

wherein

$R^1$ is a $C_1$- to $C_4$-alkyl radical or a mixture of such radicals and
x has a value of from 2 to 6,

(B) from 5 to 80% by weight, based on the weight of the total composition, of one or more alkoxy glycol components having the general formula (IV)

$$R^2\text{-O-}(CH_2CH_2\text{-O})_y\text{-H} \qquad \text{(IV)}$$

wherein

$R^2$ is a $C_1$- to $C_8$-alkyl radical or a mixture of such radicals and
y has a value of from 2 to 6,
$R^2$ and/or y being different or identical to $R^1$ and/or x, respectively, preferably identical,

(C) from 0.1 to 10% by weight, based on the total weight of the composition, of an additive package comprising one or more additives with corrosion inhibition action, wherein at least one of the alkanol ethoxylates $R^1$-O-($CH_2CH_2$-O-)-H in component (A) or component (B) is an alkanol ethoxylate according to Claim 8.

**15.** Borate-free brake fluids comprising

(D) from 50 to 85, preferably 60 - 82 wt.-% of alkoxy glycol according to formula (VI)

$$H_3C\text{-O-}(CH_2\text{-}CH_2\text{-O})_p\text{-H} \qquad \text{(VI)}$$

wherein p is a number from 2 to 5, with the proviso that in at least 30 wt.-% of all compounds according to formula (VI) p is 3, and
(E) from 1 to 15, preferably 1.5 to 10 wt.-% of alkoxy glycol according to formula (VII)

$$R^3\text{-O-}(CH_2\text{-}CH_2\text{-O})_q\text{-H} \qquad \text{(VII)}$$

wherein
$R^3$ is a $C_2$ to $C_8$ alkyl residue,
q is a number from 2 to 6,
with the proviso that in at least 65 wt.-% of all compounds according to formula (VII) q is 3, and
(F) from 6 to 35, preferably 10 - 25 wt.-% of at least one compound according to formula (VIII)

$$H\text{-O-}(CH_2\text{-}CH_2\text{-O})_r\text{-H} \qquad \text{(VIII)}$$

wherein r is a number of 2 or higher, with the proviso that in at least 80 wt.-% of all compounds according to formula (VIII) r is 2 or 3,
(G) at least one additive, selected from the group consisting of corrosion inhibitor, amines, stabilizers, defoamers and lubricants,
the fluid comprising at most 3 wt.-% of an ester between boric acid and a glycol or polyglycol compound,

wherein components (D) and/or (E) are alkanol ethoxylate according to Claim 8 and/or component (F) is an ethylene glycol according to Claim 6.

**16.** Silicon-containing brake fluids comprising

(D) at least one alkoxy glycol according to formula (VI)

$$H_3C\text{-}O\text{-}(CH_2\text{-}CH_2\text{-}O)_p\text{-}H \qquad (VI)$$

wherein p is a number from 2 to 5, with the proviso that in at least 30 wt.-% of all compounds according to formula (VI) p is 3, and
(E) at least one alkoxy glycol according to formula (VII)

$$R^3\text{-}O\text{-}(CH_2\text{-}CH_2\text{-}O)_q\text{-}H \qquad (VII)$$

wherein

$R^3$ is a $C_2$ to $C_8$ alkyl residue,
q is a number from 2 to 6,
with the proviso that in at least 65 wt.-% of all compounds according to formula (VII) q is 3, and

(F) optionally at least one compound according to formula (VIII)

$$H\text{-}O\text{-}(CH_2\text{-}CH_2\text{-}O)_r\text{-}H \qquad (VIII)$$

wherein r is a number of 2 or higher, with the proviso that in at least 80 wt.-% of all compounds according to formula (VIII) r is 2 or 3,
(G) at least one additive, selected from the group consisting of corrosion inhibitor, amines, stabilizers, defoamers and lubricants,
the fluid comprising at most 3 wt.-% of an ester between boric acid and a glycol or polyglycol compound,
(J) at least one organic silicon compound of formula (IX)

$$R^4_s\text{-}Si(\text{-}[\text{-}O\text{-}CH_2\text{-}CH_2\text{-}]_t\text{-}O\text{-}R^5)_{4-s}$$

wherein

s is a positive integer 1, 2 or 3, preferably 1 or 2, more preferably 2,
t is a positive integer from 2 to 4, preferably 3 or 4, more preferably 3,
$R^4$ $C_1$- to $C_4$-alkyl, preferably methyl or ethyl, more preferably methyl and
$R^5$ $C_1$- to $C_4$-alkyl, preferably methyl or n-butyl, more preferably methyl
comprising

(D) from 24 to 45 wt%, preferably 24 to 40, more preferably 24 to 35, and especially 24 to 30 wr%,
(E) from 5 to 20, preferably 7 to 15 wt%,
(F) 0 to 10, preferably 0 to 5 wt%,
(G) more than 0 to 5 wt%, preferably 1 to 4 wt%, and
(J) from 40 to 55, preferably 43 to 55, even more preferably 45 to 53, and especially 47 to 52 wt%,
with the proviso that the components (D) to (G) and (J) always add up to 100 wt%,
wherein components (D) and/or (E) are alkanol ethoxylate according to Claim 8 and/or component (F) is an ethylene glycol according to Claim 6 and/or component (J) comprise alkanol ethoxylates according to Claim 8.

Europäisches Patentamt
European Patent Office
Office européen des brevets

# EUROPEAN SEARCH REPORT

Application Number
EP 23 20 6933

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | WO 2021/089183 A1 (LINDE GMBH [DE]) 14 May 2021 (2021-05-14)<br>* page 6, lines 20-24; figure *<br>* page 4, lines 11-16 *<br>----- | 6,7<br><br>6-9, 12-16 | INV.<br>C07C29/10<br>C07C31/20<br>C07C41/03<br>C07C43/11<br>C08G65/08<br>C25B1/04<br>C07C1/20<br>C07C11/04<br>C07D304/04 |
| X<br><br>Y | CN 114 685 240 A (DONGHUA ENGINEERING TECH CO LTD; HIGH CHEMICAL JOINT STOCK AGENCY) 1 July 2022 (2022-07-01)<br>* paragraphs [0003], [0017] - [0019] *<br>----- | 6,7<br><br>6-9, 12-16 | |
| X<br><br>A | FELLOWS S K ET AL: "Availability of large quantities of low-deuterium hydrogen, and possible uses", INTERNATIONAL JOURNAL OF HYDROGEN ENERGY, ELSEVIER, AMSTERDAM, NL, vol. 6, no. 1, 1 January 1981 (1981-01-01), pages 67-71, XP025591252, ISSN: 0360-3199, DOI: 10.1016/0360-3199(81)90098-7 [retrieved on 1981-01-01]<br>* pages 68-69 *<br>----- | 10,11<br><br>1 | TECHNICAL FIELDS SEARCHED (IPC) |
| X<br><br>A | US 2017/137287 A1 (LIU HONGJIAN [CN] ET AL) 18 May 2017 (2017-05-18)<br>* paragraph [0008] *<br>----- | 10,11<br><br>1 | C07C<br>C08G<br>C07D<br>C25B |
| Y | US 2002/132864 A1 (SEARLE RONALD G [US]) 19 September 2002 (2002-09-19)<br>* paragraphs [0002], [0089]; figure *<br>-----<br>-/-- | 1-9, 12-16 | |

The present search report has been drawn up for all claims

1

| Place of search | Date of completion of the search | Examiner |
|---|---|---|
| The Hague | 12 April 2024 | Kardinal, Siegmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 23 20 6933

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ROODE-GUTZMER QUIRINA I. ET AL: "Renewable Methanol Synthesis", CHEMBIOENG REVIEWS, vol. 6, no. 6, 3 December 2019 (2019-12-03), pages 209-236, XP055809052, Hoboken, USA ISSN: 2196-9744, DOI: 10.1002/cben.201900012 Retrieved from the Internet: URL:https://onlinelibrary.wiley.com/doi/full-xml/10.1002/cben.201900012> * page 214 - page 228 * ----- | 1-9, 12-16 | |
| Y | WO 2022/043303 A1 (BASF SE [DE]) 3 March 2022 (2022-03-03) * claims; examples * ----- | 1-9,12, 13 | |
| Y | WO 2013/171052 A1 (BASF SE [DE]; BASF SCHWEIZ AG [CH]) 21 November 2013 (2013-11-21) * claims; examples * ----- | 1-9,14 | TECHNICAL FIELDS SEARCHED (IPC) |
| Y | WO 2021/213693 A1 (CLARIANT INT LTD [CH]) 28 October 2021 (2021-10-28) * claims; examples * ----- | 1-9,15 | |
| Y | "New Functional Fluids", RESEARCH DISCLOSURE, KENNETH MASON PUBLICATIONS, HAMPSHIRE, UK, GB , vol. 694, no. 49 1 January 2022 (2022-01-01), XP007149976, ISSN: 0374-4353 Retrieved from the Internet: URL:https://www.researchdisclosure.com/database/RD694049 [retrieved on 2022-01-17] * examples * ----- | 1-9,16 | |

The present search report has been drawn up for all claims

1

| Place of search | Date of completion of the search | Examiner |
|---|---|---|
| The Hague | 12 April 2024 | Kardinal, Siegmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 23 20 6933

This annex lists the patent family members relating to the patent documents cited in the above-mentioned European search report. The members are as contained in the European Patent Office EDP file on
The European Patent Office is in no way liable for these particulars which are merely given for the purpose of information.

12-04-2024

| Patent document cited in search report | Publication date | Patent family member(s) | Publication date |
|---|---|---|---|
| WO 2021089183 A1 | 14-05-2021 | DE 102019007672 A1 | 06-05-2021 |
| | | WO 2021089183 A1 | 14-05-2021 |
| CN 114685240 A | 01-07-2022 | NONE | |
| US 2017137287 A1 | 18-05-2017 | CA 2949279 A1 | 03-12-2015 |
| | | TW M513734 U | 11-12-2015 |
| | | TW 201545972 A | 16-12-2015 |
| | | US 2017137287 A1 | 18-05-2017 |
| | | WO 2015180592 A1 | 03-12-2015 |
| US 2002132864 A1 | 19-09-2002 | AU 9666001 A | 15-05-2002 |
| | | US 6495609 B1 | 17-12-2002 |
| | | US 2002132864 A1 | 19-09-2002 |
| | | WO 0236532 A2 | 10-05-2002 |
| WO 2022043303 A1 | 03-03-2022 | BR 112023002916 A2 | 21-03-2023 |
| | | CA 3190928 A1 | 03-03-2022 |
| | | CN 115989601 A | 18-04-2023 |
| | | EP 3960834 A1 | 02-03-2022 |
| | | ES 2958937 T3 | 16-02-2024 |
| | | HU E063553 T2 | 28-01-2024 |
| | | JP 2023538745 A | 11-09-2023 |
| | | KR 20230055398 A | 25-04-2023 |
| | | RS 64495 B1 | 29-09-2023 |
| | | WO 2022043303 A1 | 03-03-2022 |
| WO 2013171052 A1 | 21-11-2013 | BR 112014028179 A2 | 27-06-2017 |
| | | CA 2871544 A1 | 21-11-2013 |
| | | CN 104302747 A | 21-01-2015 |
| | | EP 2850163 A1 | 25-03-2015 |
| | | ES 2728662 T3 | 28-10-2019 |
| | | JP 2015516495 A | 11-06-2015 |
| | | KR 20150008189 A | 21-01-2015 |
| | | WO 2013171052 A1 | 21-11-2013 |
| WO 2021213693 A1 | 28-10-2021 | AU 2020343995 A1 | 11-11-2021 |
| | | CA 3115303 A1 | 23-10-2021 |
| | | CN 113853422 A | 28-12-2021 |
| | | EP 3938479 A1 | 19-01-2022 |
| | | ES 2948341 T3 | 08-09-2023 |
| | | HR P20230836 T1 | 10-11-2023 |
| | | HU E062010 T2 | 28-09-2023 |
| | | JP 7157247 B2 | 19-10-2022 |
| | | JP 2022539932 A | 14-09-2022 |
| | | KR 20210132636 A | 04-11-2021 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO.

EP 23 20 6933

This annex lists the patent family members relating to the patent documents cited in the above-mentioned European search report. The members are as contained in the European Patent Office EDP file on
The European Patent Office is in no way liable for these particulars which are merely given for the purpose of information.

12-04-2024

| Patent document cited in search report | Publication date | Patent family member(s) | Publication date |
|---|---|---|---|
| | | PL 3938479 T3 | 23-10-2023 |
| | | US 2023340359 A1 | 26-10-2023 |
| | | WO 2021213693 A1 | 28-10-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 103848399 A
- EP 2023060570 W
- US 3629343 A
- GB 1777877 A
- DE 2615595
- US 4160116 A
- US 4233221 A
- US 6156720 A
- DD 203735
- DD 203734
- WO 9729146 A
- WO 9803571 A
- WO 0014143 A
- WO 9944739 A
- US 20080161509 A1
- US 20080207939 A1
- US 3462525 A
- US 3420875 A
- US 3524864 A
- EP 310875 A
- EP 356725 A
- EP 700985 A
- US 4877416 A
- EP 2023061271 W
- DE 1948794 A
- US 6558569 B
- EP 2023063884 W


### Non-patent literature cited in the description


- **S. KUMAR** ; **V. HIMABINDU**. *Material Science for Energy Technologies*, 2019, vol. 2, 4442-4454
- **H. A. MILLER et al.** *Sustainable Energy Fuels*, 2020, vol. 4, 2114-2133
- **K. KAMLUNGSUA et al.** *FUEL CELLS*, 2020, vol. 20 (6), 644-649
- **KOYTSOUMOA et al.** *The Journal of Supercritical Fluids*, February 2018, vol. 132, 3-16
- **CHEN, LACKNER et al.** Sorbents for the Direct Capture of CO2 from Ambient Air. *Angew. Chem. Int. Ed.*, 2020, vol. 59, 6984-7006
- **KOMMALAPATI et al.** *Energy Technol.*, 2017, vol. 5, 822-833
- **R. GUIL-L6PEZ**. *Materials*, 2019, vol. 12, 3902
- **KRISTIAN STANGE-LAND** ; **HAILONG LI** ; **ZHIXIN YU**. *Energy, Ecology and Environment*, 2020, vol. 5, 272-285
- **ADAM CHAN**. Ethylene. *Nexant, TECH*, July 2018, 100-109
- Ethylene Oxide. **MIA MONCONDUIT** ; **KAREN JOBES**. IHS Markit, Chemical Economics Handbook. 22 December 2020, 14-16
- Ullmann's Encyclopedia of Industrial Chemistry. 1994, vol. A25, 779-783
- **D. STÖVER et al.** *Journal of Power Sources*, 15 November 2012, vol. 218, 157-162
- **C.C. KLEPPER** ; **T.M. BIEWER** ; **U. KRUEZI** ; **S. VARTANIAN** ; **D. DOUAI** ; **D.L. HILLIS** ; **C. MARCUS**. Extending helium partial pressure measurement technology to JET DTE2 and ITER. *Rev. Sci. Instrum.*, 2016, vol. 87 (11)
- **S. DAVIES** ; **J.A. REES** ; **D.L. SEYMOUR**. Threshold ionisation mass spectrometry (TIMS); A complementary quantitative technique to conventional mass resolved mass spectrometry. *Vacuum*, 2014, vol. 101, 416-422
- **W.A. BRANDT**. RAPID COMMUNICATIONS IN MASS SPECTROMETRY Rapid Commun. *Mass Spectrom.*, 1999, vol. 13, 1226-1230